# EUROPEAN PATENT APPLICATION

(11) **EP 2 172 444 A1**
(43) Date of publication of application: **07.04.2010**
(21) Application number: 08777270.3
(22) Date of filing: 17.06.2008
(51) Int. Cl.: C07C 217/56, A61K 31/428, A61K 31/437, A61K 31/44, A61K 49/00, A61P 25/00, A61P 25/14, A61P 25/16, A61P 25/28, A61P 31/12, C07C 309/73, C07D 213/64, C07D 277/66, C07D 471/04, C07B 59/00

(54) **PET PROBE HAVING ALKOXY GROUP SUBSTITUTED BY FLUORINE AND HYDROXY GROUP**

(30) Priority: 04.07.2007 JP 2007176368
(71) Applicant: Tohoku University, Aoba-ku Sendai-shi Miyagi 980-8577 (JP)
(72) Inventor: KUDO, Yukitsuka, Sendai-shi Miyagi 980-8577 (JP); FURUMOTO, Syozo, Sendai-shi Miyagi 980-8577 (JP); OKAMURA, Nobuyuki, Sendai-shi Miyagi 980-8577 (JP)
(74) Representative: Zwicker, Jörk
(86) International application number: PCT/JP2008/061032
(87) International publication number: WO 2009/004914

(57) **Abstract**

Disclosed are: a PET probe compound having an alkoxy group substituted by a fluorine and a hydroxy group, which is useful for the early diagnosis of a conformational disease; a pharmaceutical composition for the treatment and/or prevention of a conformational disease, which comprises the compound; and others.

## Description

### TECHNICAL FIELD

The present invention relates to a diagnostic probe for conformation disease, in particular to an imaging diagnostic probe, and more particularly, to a probe labeled with a positron emitter, and to a composition for imaging diagnosis that comprises the probe. Further, the invention relates to a pharmaceutical composition, for example, for detection/staining of amyloid β protein and neurofibrillary tangle in a brain material, and for example, for detection/staining of senile plaque in the brain of Alzheimer disease patients, and for prevention and/or treatment of conformation disease. The invention also relates to a composition for diagnosis of conformation disease that comprises the above-mentioned probe compound.

### BACKGROUND ART

Disorders with deposition of a β sheet structured protein that is intrinsic to conformation disease include various diseases characterized by deposition of insoluble fibrillary protein in various organs and tissues of a body. These diseases include Alzheimer disease, prion disease, Lewy body disease, Parkinson disease, Huntington disease, spinobulbar muscular atrophy, dentatorubral-pallidoluysian atrophy, spinocerebellar ataxia, Machado-Joseph disease, amyophic lateral sclerosis (ALS), Down syndrome, Pick disease, FTDP-17 (frontotemporal dementia and parkinsonism linked to chromosome 17), LNTD (limbic neurofibrillary tangle dementia), sudanophilic leukodystrophy, amyloidosis, etc.

Of those, Alzheimer disease (AD) is at present considered as one of most incurable diseases, and accurate early diagnosis is desired for it. Alzheimer disease is a disease characterized by progressive dementia occurring essentially in the presenile stage to the senile stage. From the pathological viewpoint, the disease is characterized by entire cerebral atrophy, extreme degeneration and omission of neurons and appearance of neurofibrillary tangle and senile plaque. It is known that the most significant risk factor of dementia such as typically Alzheimer disease is aging. Accordingly, the increase in the number of the case patients with the increase in the senile population is remarkable especially in Japan, America and European countries that are in aging society, and the medical cost for the disease has brought about a crisis of the medical system in these countries.
In our country, the number of Alzheimer disease patients is estimated at about 1,000,000, and with the increase in the senile population in future, the number of the patients will surely increase. The cost for one Alzheimer disease patient inclusive of care expense will be from 1,000,000 yen to 3,000,000 yen/year, and therefore, our country would have already paid a social economic cost of from 1,000,000,000,000 yen to 3,000,000,000,000 yen. Medical treatment of Alzheimer disease before the actual development of the symptom of the disease or in the stage thereof as early as possible could bring about a great medical economic effect, and it is now a global common sense.

At present, various methods are known for diagnosis of Alzheimer disease. In our country, generally employed is a method of quantitatively detecting the reduction in the cognitive function of an individual that may have suffered from Alzheimer disease, such as a Hasegawa method, ADAS, MMSE or the like; but rarely and secondarily employed is an imaging diagnostic method (e.g., MRI, CT). However, these diagnostic methods are unsatisfactory for deciding the disease, and the definite diagnosis requires biopsy of the brain during the lifetime and histopathologic examination of the brain after death. Despite of diligent studies made for it, no one could make any significant progress in diagnosis of Alzheimer disease. As a result of many studies, it has become known that neurodegeneration characteristic of Alzheimer disease may begin much before the development of the first clinical symptom of the disease (in a long case, it is before about 40 years). In addition, it is known that, when the family or the clinicians around the patient of the disease have noticed the first clinical symptom of the disease, then the intracerebral pathologic image of the patient has already advanced to an irreparable state. In consideration of the progressive characteristic of the disease symptom and of the significant increase in the number of the disease patients, the necessity and the significance of accurate early stage diagnosis of Alzheimer disease is extremely great.

The histopathologic image of Alzheimer disease is characterized by two typical cardinal signs. They are senile plaque and neurofibrillary tangle. The essential constitutive component of the former is a β sheet structured amyloid β (Aβ) protein; and that of the latter is an hyperphosphorylated amyloid β protein. The definite diagnosis of Alzheimer disease is based on the expression of these pathologic characteristics in a patient's brain.

Amyloid β protein is characteristic of conformation disease that includes Alzheimer disease, and the two have close relation to each other. Accordingly, detection of a β sheet structured amyloid β protein as a marker in a body, especially in a brain is one important method for diagnosis of conformation disease, especially Alzheimer disease. Searches for substances capable of specifically binding to intracorporeal, especially intracerebral amyloid β protein to stain it have heretofore been made for the purpose of diagnosis of a disease with amyloid deposition such as typically Alzheimer disease. As such substances, known are Congo red (see Non-Patent Reference 1), Thioflavin S (see Non-Patent Reference 2), Thioflavin T (Non-Patent Reference 3) and Crysamine G and its derivatives (see Patent Reference 1 and Patent Reference 2). However, these have a lot of problems in point of their binding specificity to amyloid β protein, blood-brain barrier permeability, solubility and toxicity. We, the present inventors have found out various compounds characterized by high specificity to amyloid β protein, great blood-brain barrier permeability and solubility and less toxicity (see Patent Reference 3, Patent Reference 4, Patent Reference 5, Patent Reference 6 and Patent Reference 7).

A disease is known, which is caused by an intracerebral protein itself having a β sheet structure. It is considered that, in Alzheimer disease, amyloid β protein and tau protein may have a β sheet structure and the proteins themselves may be a cause of the disease or a part of the cause of the disease. Yankner, et al. reported for the first time that, when amyloid β protein is made to have a β sheet structure, then it exhibits neuron toxicity (see Non-Patent Reference 4). After that, many replication studies for it have been made, and have confirmed that the β sheet structured amyloid β protein has neuron toxicity. In that manner, the β sheet structured amyloid β protein and tau protein have neuron toxicity, and therefore, it may be suggested that a compound capable of inhibiting the cytotoxicity could be a remedial drug for a disease, of which the cause or a part of the cause is the β sheet structured protein itself, or that is, conformation disease such as Alzheimer disease. At present, however, the development of such a remedial drug could not bring about a sufficient result.

Accordingly, the necessity is increasing for a compound having high specificity to amyloid β protein for diagnosis of conformation disease such as typically Alzheimer disease, for a staining agent specific to amyloid β protein, and for treatment and prevention of conformation disease.

Another histopathologic cardinal sign of Alzheimer disease comprises neurofibrillary tangle and its essential constitutive component, hyperphosphorylated tau protein, but in general, it is considered that these may be expressed later than amyloid β protein. However, it is considered that neurofibrillary tangle may well correlate to the degree of dementia as compared with amyloid β protein (see Non-Patent Reference 5 and Non-Patent Reference 6).
Apart from Alzheimer disease, disorders characterized by the cardinal sign of intracerebral deposition tau protein (tauopathy) are Pick disease and progressive supranuclear palsy (PSP). Conformation disease also includes these diseases.

To that effect, tau protein is characteristic of the disease with deposition of tau protein that includes Alzheimer disease, and it has close relation to the disease. Accordingly, the detection of intracorporeal, especially intracerebral β sheet structured tau protein as a marker is one important method for diagnosis of diseases with tau deposition, especially Alzheimer disease.

A method for quantitatively determining the tau level in a body, especially in a cerebrospinal fluid for the purpose of diagnosis of tau deposition-associated diseases such as typically Alzheimer disease has been reported by a few groups (see Non-Patent Reference 7 and Non-Patent Reference 8). However, any probe for *in-vivo* noninvasive quantitative determination of tau is not known at all in the world.

Accordingly, a necessity is increasing for a compound having high specificity to neurofibrillary tangle, for diagnosis and treatment of a disease of which the cause or a part of the cause is neurofibrillary tangle such as typically Alzheimer disease or for staining neurofibrillary tangle.

Compounds with high specificity for amyloid-beta proteins and neurofibrillary tangle have been reported (see patent document 8, patent document 9, patent document 10, and patent document 11). When these compounds are used in-vivo, in particular, in the body of a human patient, it is preferable that the compounds provide extremely low or no bone accumulation. Consequently, search of compounds which provide extremely low or no bone accumulation, and which can be used as conformation disease diagnosis probe is necessary.
Patent Reference 1: PCT/US96/05918
Patent Reference 2: PCT/US98/07889
Patent Reference 3: Japanese Patent Application 2000-080082
Patent Reference 4: Japanese Patent Application 2000-080083
Patent Reference 5: Japanese Patent Application 2001-076075
Patent Reference 6: PCT/JP01/02204
Patent Reference 7: PCT/JP01/02205
Patent Reference 8: PCT/JP03/07183
Patent Reference 9: PCT/JP03/15269
Patent Reference 10: PCT/JP03/15229
Patent Reference 11: PCT/JP2004/01546
Non-Patent Reference 1: Puchtler et al., Journal of Histochemistry and Cytochemistry, Vol. 10, p. 35, 1962
Non-Patent Reference 2: Puchtler et al., Journal of Histochemistry and Cytochemistry, Vol. 77, p. 431, 1983
Non-Patent Reference 3: Le Vine, Protein Science, Vol. 2, pp. 404-410, 1993
Non-Patent Reference 4: Yankner et al., Science, Vol. 245, pp. 417-420, 1989
Non-Patent Reference 5: Braak H. and Braak E., Acta Neuropathol., Vol. 82, pp. 239-259, 1991
Non-Patent Reference 6: Wischik et al., Neurobiology of Alzheimer's Disease, pp. 103-206, Oxford University Press, Oxford, 2001
Non-Patent Reference 7: Ishiguro et al., Neurosci. Lett., Vol. 270, pp. 81-84, 1999
Non-Patent Reference 8: Itoh et al., Ann. Neurol., Vol. 50, pp. 150-156, 2001

### DISCLOSURE OF THE INVENTION

### PROBLEMS THAT THE INVENTION IS TO SOLVE

In view of the above-mentioned situation, the present invention provides a substance which has high specificity for amyloid-beta proteins and/or neurofibrillary tangle, has high blood-brain barrier permeability, and provides low or no bone accumulation, and which can be used for conformation disease diagnostic probe. In addition, the present invention provides such labeled substance used for conformation disease diagnostic imaging probe as well as such diagnostic imaging diagnostic compositions and kits that include a probe. Furthermore, the present invention provides detection and/or staining method of amyloid-beta proteins and neurofibrillary tangle in the brain material, kits for that purpose, as well as pharmaceutical compositions for prevention and/or treatment of conformation disease. In addition, the present invention provides compounds useful for early diagnosis of conformation diseases and diagnostic imaging compositions that comprise the compounds.

### MEANS FOR SOLVING THE PROBLEM

The present inventors have intensively studied to solve the above-mentioned problems, and found that a compound selected from the group consisting of formula (I): 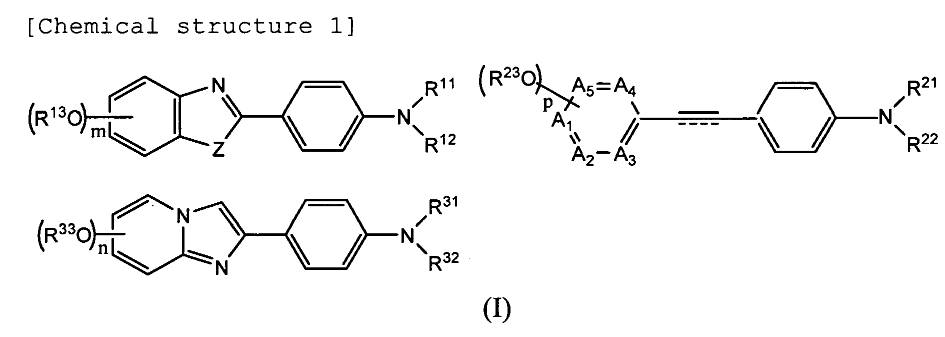 and
[wherein: any one of R11, R12, or R13 is a group represented by formula (II):

[Chemical structure 2] -X-F (II)

(wherein X is an alkyl group of 3 through 10 carbons substituted by 1 through 3 hydroxy groups), and
remaining two of R11, R12 or R13 are hydrogen atoms or lower alkyl groups, or
in case that R13 is a group represented by above-mentioned formula (II), R11, R12 and nitrogen atoms bonded thereto, together, form 3-8 membered nitrogen-containing aliphatic cyclic group,
m is 1, or in case that either of R11 or R12 is a group represented by above-mentioned formula (II), m may be 0,
Z represents an oxygen atom or sulfur atom,
any one of R21, R22 or R23 is a group represented by formula (II):

[Chemical structure 3] -X-F (II)

(wherein X has the above-mentioned meaning), and
remaining two of R21, R22 or R23 are hydrogen atoms or lower alkyl groups, or
in case that R23 is a group represented by above-mentioned formula (II), R21, R22 and nitrogen atoms bonded thereto, together, form 3-8 membered nitrogen-containing aliphatic cyclic group,
n is 1, or in case that either of R21 or R22 is a group represented by above-mentioned formula (II), n may be 0, and 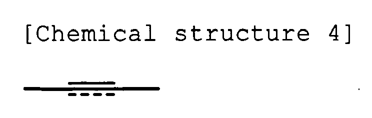 indicates 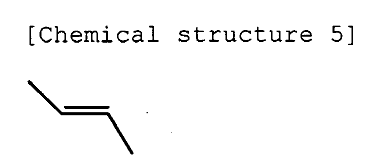 or 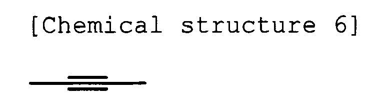 ,and any one of R31, R32 or R33 is a group represented by formula (II):

[Chemical structure 7] -X-F (II)

(wherein X has the above-mentioned meaning), and
remaining two of R31, R32 or R33 are hydrogen atoms or lower alkyl groups, or
in case that R33 is a group represented by above-mentioned formula (II), R31, R32 and nitrogen atoms bonded thereto, together, form 3-8 membered nitrogen-containing aliphatic cyclic group,
n is 1, or in case that either of R31 or R32 is a group represented by above-mentioned formula (II), n may be 0, and
one or two of A1 through A5 is a nitrogen atom and the remaining three or four are CH], pharmaceutically acceptable salt or solvate thereof can be used as a diagnostic probe of conformation disease which has high specificity to beta-amyloid proteins and/or neurofibrillary tangle, as well as high blood-brain barrier permeability, and extremely small or no bone accumulation, and reached to achieve the present invention.

The compound which has an -O- alkyl group or -N-alkyl group (the alkyl group is substituted with a halogen atom and hydroxyl group and further substituted with a fluorine atom) at the terminal provides low or extremely low bone accumulation. It is one of the characteristics of the present invention to contain a compound with low or extremely low bone accumulation.
The present invention is to provide a compound, pharmaceutically acceptable salt or solvate thereof with higher safety than conventional amyloid PET probe by having an -O- alkyl group or -N-alkyl group (the alkyl group is substituted with a halogen atom and hydroxyl group and further substituted with a fluorine atom) at the terminal of the compounds represented by formula (I).
Therefore, the compound according to the present invention provides extremely high safety. In addition, because the compound according to the present invention specifically and distinctly stains beta-amyloid proteins, it can be said that the compound enables accurate early diagnosis particularly of Alzheimer's Disease, Down syndrome. etc. Furthermore, the compound according to the present invention has also high blood-brain barrier permeability, that is, high blood-brain barrier permeability. Based on these characteristics, the use of the compound of the present invention makes it possible to achieve noninvasive early diagnosis in-vivo, in particular, in human patients.

As a drug for a PET probe to aid diagnosis of amyloid, for example, in WO2007/002540, ethylene glycol or polyethylene glycol with a labeled terminal is disclosed. However, in the present specifications, no specific disclosure is given with respect to what kind of terminal group is superior as a terminal group with low or nearly free of bone accumulation, and it is disclosed that using polyethylene glycol for the labeled terminal group can lower the lipid solubility.

### EFFECT OF THE INVENTION

The present invention provides a compound with extremely high safety, which has remarkably high specificity for beta-amyloid proteins and/or neurofibrillary tangle, and high blood-brain barrier permeability, low or no bone accumulation. In addition, the present invention provides a compound with extremely high safety, which has remarkably high specificity for beta-amyloid proteins and/or neurofibrillary tangle, and high blood-brain barrier permeability, low or practically no bone accumulation. Therefore, it is possible to diagnose, treatment and/or prevention conformation diseases by the use of the compound of the present invention. In addition, according to the present invention, diagnostic imaging of conformation diseases, in particular, diagnostic imaging by the use of PET is possible. therefore, the present invention enables accurate early diagnosis, effective treatment, and prevention of conformation diseases.

### BRIEF DESCRIPTION OF THE DRAWINGS

[Fig. 1]

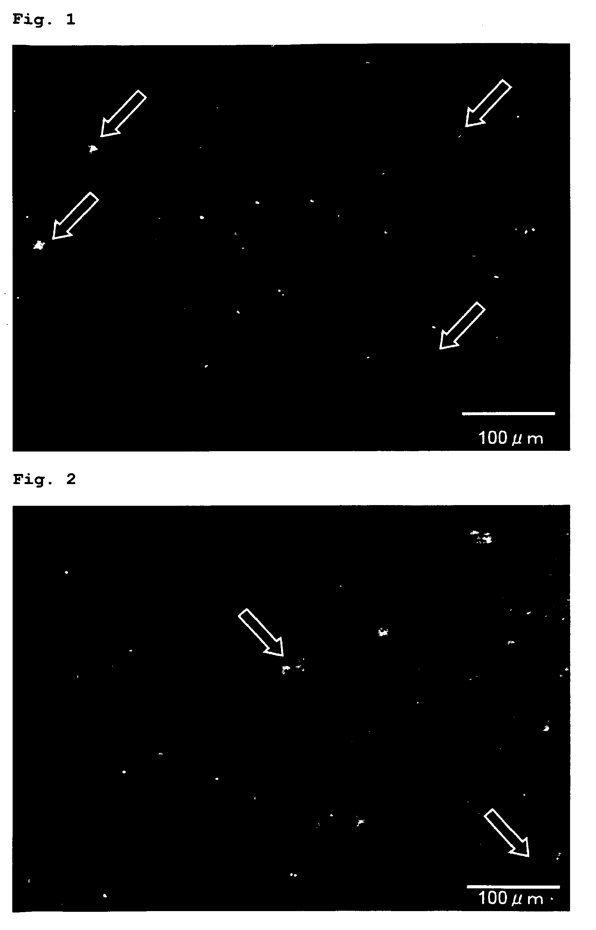

   Fig. 1 is a THK-837 staining image in a brain section of a patient with Alzheimer's Disease. The outline arrow mark shows beta-amyloid proteins.
[Fig. 2]
   Fig. 2 is a THK-853 staining image in a brain section of a patient with Alzheimer's Disease. The outline arrow mark shows beta-amyloid proteins.

### BEST MODE FOR CARRYING OUT THE INVENTION

The compound according to the present invention is a compound, salt or solvate thereof represented by formula (I) explained as follows. In the present specifications, the "compound of the invention" includes a compound, salt or solvate thereof represented by formula (I), if not otherwise specified.

In the present specifications, the "low alkyl group" means a straight-chain or branched alkyl group of 1 through 6 carbons, and specifically includes, methyl group, ethyl group, propyl group, isopropyl group, butyl group, isobutyl group, sec-butyl group, tert-butyl group, pentyl group, isoamyl group, neopentyl group, isopentyl group, 1,1-dimethylpropyl group, 1-methylbutyl group, 2-methylbutyl group, 1,2-dimethylpropyl group, hexyl group, isohexyl group, 1-methylpentyl group, 2-methylpentyl group, 3-methylpentyl group, 1,1-dimethylbutyl group, 1,2-dimethylbutyl group, 2,2-dimethylbutyl group, 1,3-dimethylbutyl group, 2,3-dimethylbutyl group, 3,3-dimethylbutyl group, 1-ethylbutyl group, 2-ethylbutyl group, 1,2,2-trimethylpropyl group, 1-ethyl-2-methylpropyl group, and others.

In the present specifications, "beta-amyloid proteins," "β-amyloid proteins," "Aβ-proteins," "amyloid beta," "amyloid β," and "Aβ" are synonyms.

In case that any double bond exists between two rings in the compound of the present invention, isomers of both cis and trans can exist.

In addition, in case that any asymmetric carbon atom exists in the compound of the present invention, a mixture of isomers and individual isomers of these are included in the compound of the present invention.
For example, if one asymmetric carbon atom exists in the compound of the present invention, optically active compounds can be individually synthesized, or each of optical isomers can be separated by column chromatography.
In case that the optical isomers are separated by column chromatography, examples of the column used include CHIRALPAK AD (available from Daicel Chemical Industries), etc.
In addition, the solvent used may be any solvent which is generally used for separating isomers, and examples include chloroform, acetonitrile, ethyl acetate, methanol, acetone, hexane, water, etc., which may be used individually or in combination (two or more) as the solvent.

In order to still more specifically disclose the compound represented by the formula (I): 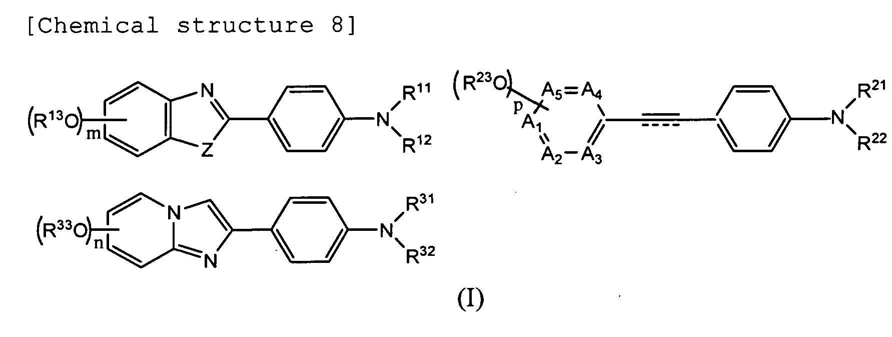 and
[wherein each variable is same as that described above], various variables used in Formula (I) will be described with reference to examples below.

Firstly, a compound represented by formula (I-1): 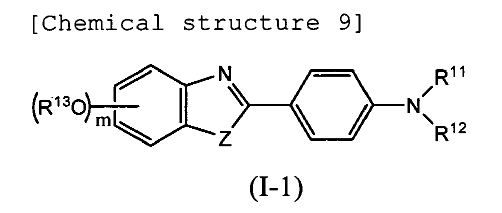 in formula (I) will be described.

Any one of R11, R12, or R13 is a group represented by formula (II):

[Chemical structure 10] -X-F (II)

and remaining two of R11, R12 or R13 are hydrogen atoms or lower alkyl groups, or
in case that R13 is a group represented by above-mentioned formula (II), R11, R12 and nitrogen atoms bonded thereto, together, form 3-8 membered nitrogen-containing aliphatic cyclic group.

X in formula (II) is an alkyl group of 3 through 10 carbons substituted by 1 through 3 hydroxy groups.
The alkyl group of 3 through 10 carbons may be either a straight-chain or branched alkyl group but preferably an alkyl group of 3 through 7 carbons, and more preferably an alkyl group in which the carbon atom adjacent to the carbon atom to which a fluorine atom is bonded is a secondary carbon atom and at the same time, a hydroxyl group is bonded to the relevant secondary carbon atom.

As a group represented by formula II, in particular, for example, a group selected from the group consisting of formula (III): 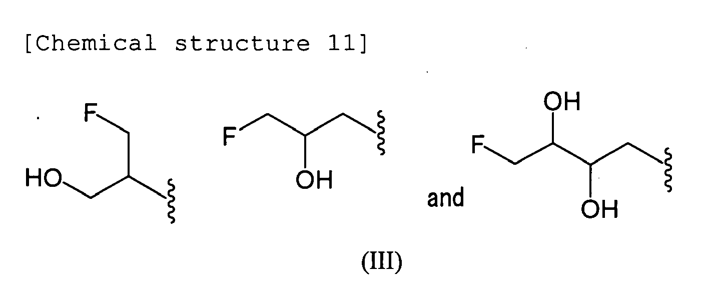 [wherein, 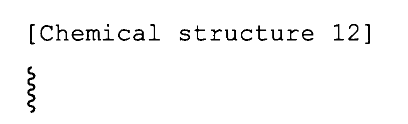 shows the bonded portion with an oxygen atom or nitrogen atom], and of these, preferably a group represented by formula (III-1) : 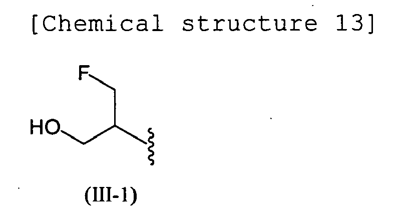 [wherein, 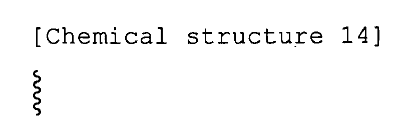 is the same as above] or a group represented by formula (III-2): 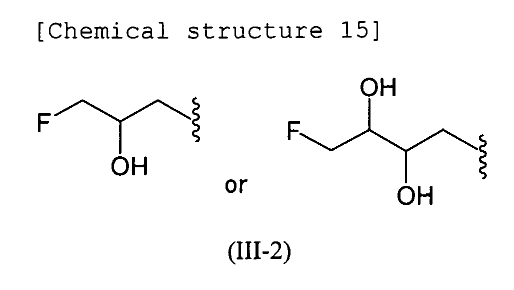 [wherein, 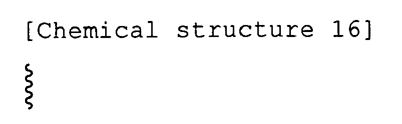 is the same as above] is exemplified.

In case that R13 is a group represented by above-mentioned formula (II), R11, R12 and nitrogen atoms bonded thereto, together, form 3-8 membered nitrogen-containing aliphatic cyclic group. Furthermore, one of the carbon atoms composing the nitrogen-containing aliphatic cyclic groups may be replaced by a nitrogen atom, sulfur atom, or oxygen atom, and specific examples of the nitrogen-containing aliphatic cyclic groups include 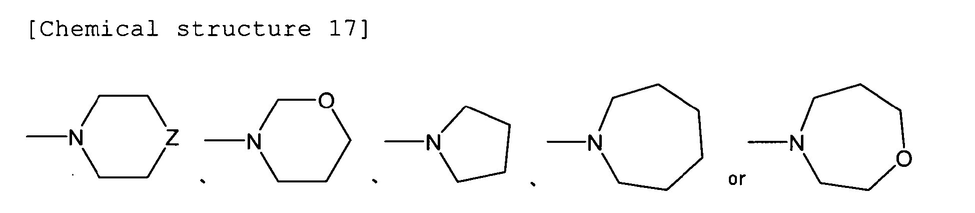 [wherein, Z represents O, S, CH₂ or NR^{e} and R^{e} represents hydrogen or C₁₋₄ alkyl group], and of these, a morpholino group is preferable.

m is 1, or in case that either of R11 or R12 is a group represented by above-mentioned formula (II), m may be 0.

Z represents an oxygen atom or sulfur atom, however it is preferable that Z is a sulfur atom.

Next discussion will be made on the compound represented by formula (I-2) of formula (I):

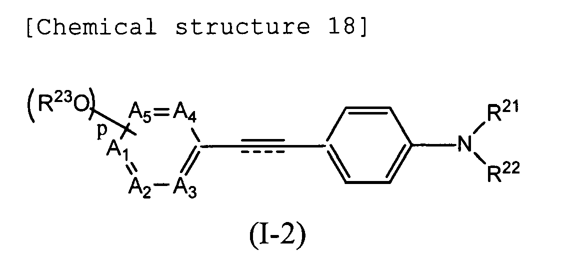

In case that any one of R21, R22, or R23 is a group represented by formula (II):

[Chemical structure 19] -X-F (II)

and remaining two of R21, R22 or R23 are hydrogen atoms or lower alkyl groups, or
in case that R23 is a group represented by above-mentioned formula (II), R21, R22 and nitrogen atoms bonded thereto, together, form 3-8 membered nitrogen-containing aliphatic cyclic group.

X in formula (II) is an alkyl group of 3 through 10 carbons substituted by 1 through 3 hydroxy groups.
The alkyl group of 3 through 10 carbons may be either a straight-chain or branched alkyl group, however preferably an alkyl group of 3 through 7 carbons, and more preferably an alkyl group in which the carbon atom adjacent to the carbon atom to which a fluorine atom is bonded is a secondary carbon atom and at the same time, a hydroxyl group is bonded to the relevant secondary carbon atom.

As a group represented by formula II, specifically, for example, a group selected from the group consisting of formula (III): 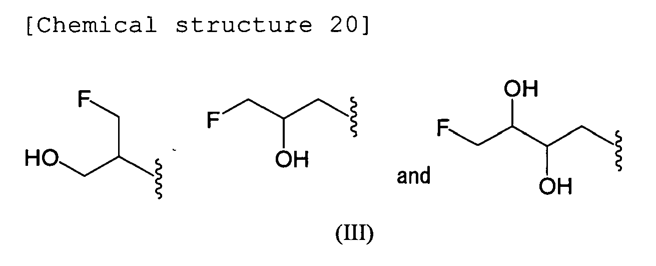 [wherein, 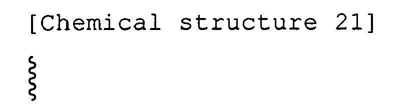 shows the bonded portion with an oxygen atom or nitrogen atom], and of these, preferably a group represented by formula (III-1): 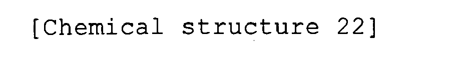 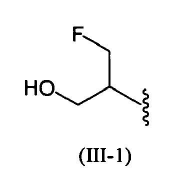 [wherein,
[Chemical formula 1] 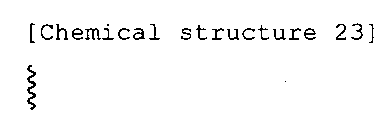 is the same as above] or a group represented by formula (III-2): 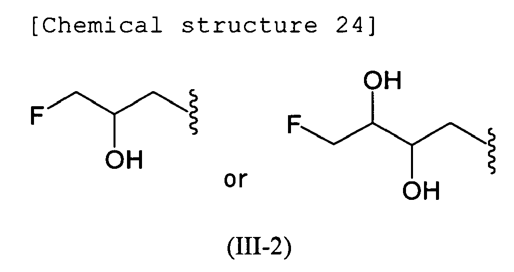 [wherein, 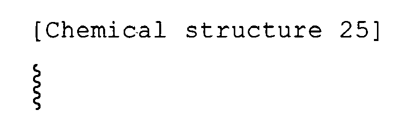 is the same as above] is exemplified.

In case that R23 is a group represented by above-mentioned formula (II), R21, R22 and nitrogen atoms bonded thereto, together, form 3-8 membered nitrogen-containing aliphatic cyclic group, and furthermore, one of the carbon atoms composing the nitrogen-containing aliphatic cyclic groups may be replaced by a nitrogen atom, sulfur atom, or oxygen atom, and specific examples of the nitrogen-containing aliphatic cyclic groups include 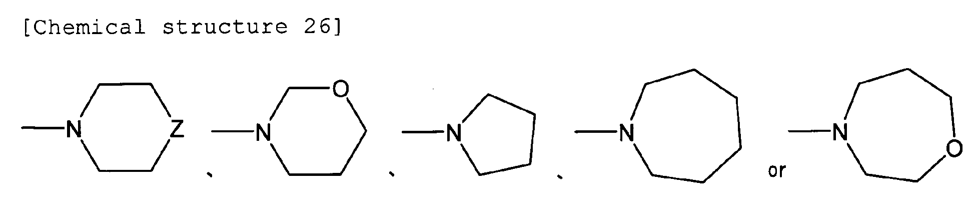 [wherein, Z represents O, S, CH₂ or NR^{e} and R^{e} represents hydrogen or C₁₋₄ alkyl group], and of these, a morpholino group is preferable.

p is 1, or in case that either of R21 or R22 is a group represented by above-mentioned formula (II), p may be 0.

All of A1 through A5 may be carbon atoms, or one or two of A1 through A5 is a nitrogen atom. Of these, it is preferable that all of A1 through A5 are carbon atoms, or one of A1 through A5 is a carbon atom.

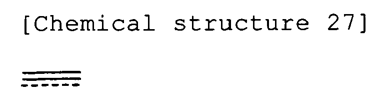

shows a double bond or a triple bond.

Next discussion will be made on the compound represented by formula (I-3) of formula (I):

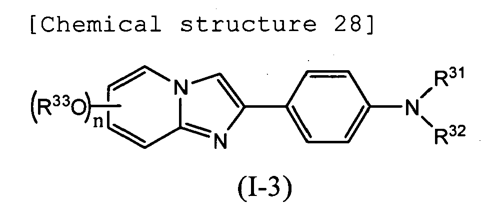

In case that any one of R31, R32, or R33 is a group represented by formula (II):

[Chemical structure 29] -X-F (II)

and remaining two of R31, R32 or R33 are hydrogen atoms or lower alkyl groups, or
in case that R33 is a group represented by above-mentioned formula (II), R31, R32 and nitrogen atoms bonded thereto, together, 3-8 membered nitrogen-containing aliphatic cyclic group.

X in formula (II) is an alkyl group of 3 through 10 carbons substituted by 1 through 3 hydroxy groups.
The alkyl group of 3 through 10 carbons may be either a straight-chain or branched alkyl group but preferably an alkyl group of 3 through 7 carbons, and more preferably an alkyl group in which the carbon atom adjacent to the carbon atom to which a fluorine atom is bonded is a secondary carbon atom and at the same time, a hydroxyl group is bonded to the relevant secondary carbon atom.

As a group represented by formula II, particularly, for example, a group selected from the group consisting of formula (III): 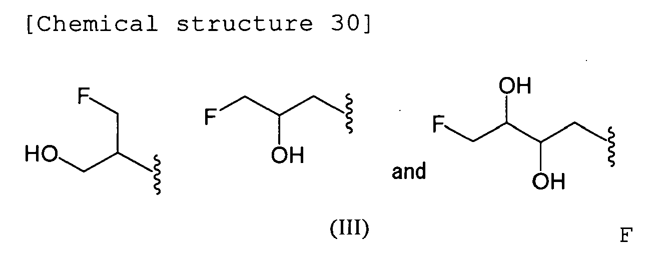 [wherein, 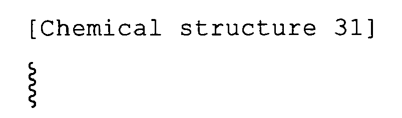 shows the bonded portion with an oxygen atom or nitrogen atom], and of these, preferably a group represented by formula (III-1): 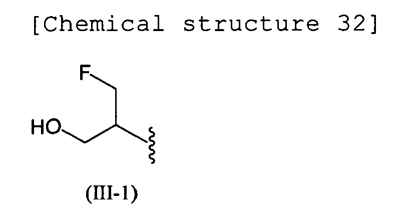 [wherein, 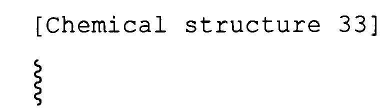 is the same as above] or a group represented by formula (III-2) : 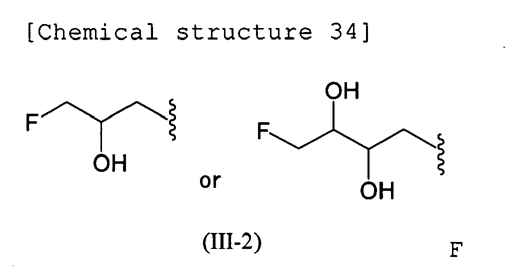 [wherein, 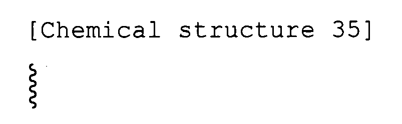 is the same as above] is exemplified.

In case that R33 is a group represented by above-mentioned formula (II), R31, R32 and nitrogen atoms bonded thereto, together, form 3-8 membered nitrogen-containing aliphatic cyclic group, and furthermore, one of the carbon atoms composing the nitrogen-containing aliphatic cyclic groups may be replaced by a nitrogen atom, sulfur atom, or oxygen atom, and specific examples of the nitrogen-containing aliphatic cyclic groups include 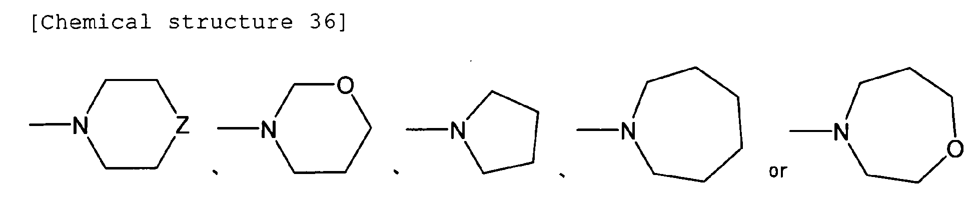 [wherein, Z represents O, S, CH₂ or NR^{e} and R^{e} represents hydrogen or C₁₋₄ alkyl group], and of these, a morpholino group is preferable.

n is 1, or in case that either of R31 or R32 is a group represented by above-mentioned formula (II), n may be 0.

These compounds, pharmaceutically acceptable salts or solvates thereof have high specificity to Aβ, and moreover, low toxicity and high intracerebral migration, and after passing specified time, a majority of the drug quickly dissolves from the brain.

Therefore, the compound according to the present invention can be used as a safe probe for diagnostic imaging of conformation diseases.

In addition, the present invention provides a compound to be used as a precursor for synthesizing a compound represented by formula (I). Those skilled in the art can easily design such a precursor from the desired compound of the present invention and can synthesize the precursor. Alternatively, such precursor may be able to be obtained by modifying commercially available compounds.

Examples of the precursor include a compound selected from the group consisting of formula (I'): 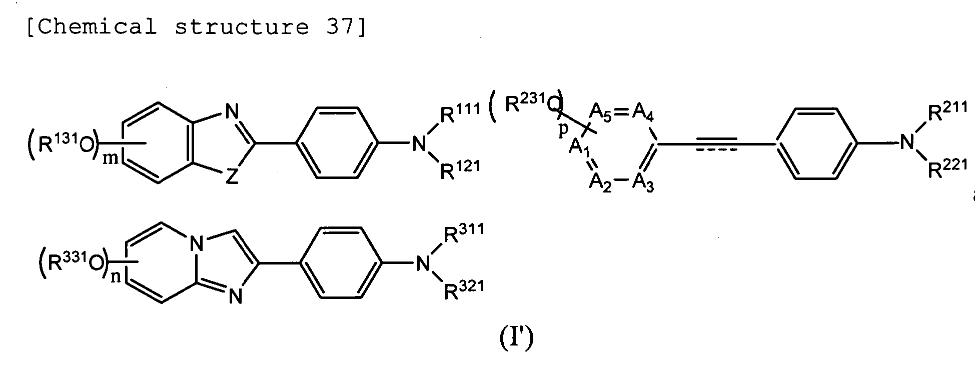 and [wherein any one of R111, R121, or R131 is a group represented by formula (II'):

[Chemical structure 38] -X-OTs (II')

(wherein X is an alkyl group of 3 through 10 carbons substituted by 1 through 3 hydroxy groups), and
remaining two of R111, R121 or R131 are hydrogen atoms or lower alkyl groups, or
in case that R131 is a group represented by above-mentioned formula (II), R111, R121 and nitrogen atoms bonded thereto, together, form 3-8 membered nitrogen-containing aliphatic cyclic group,
m is 1, or in case that either of R111 or R121 is a group represented by above-mentioned formula (II'), m may be 0,
Z represents an oxygen atom or sulfur atom,
any one of R21, R22 or R23 is a group represented by formula (II') comprising:

[Chemical structure 39] -X-OTs (II')

(wherein X has the above-mentioned meaning), and
remaining two of R211, R221 or R231 are hydrogen atoms or lower alkyl groups, or
in case that R231 is a group represented by above-mentioned formula (II'), R211, R221 and nitrogen atoms bonded thereto, together, form 3-8 membered nitrogen-containing aliphatic cyclic group,
n is 1, or in case that either of R211 or R221 is a group represented by above-mentioned formula (II'), n may be 0,
and 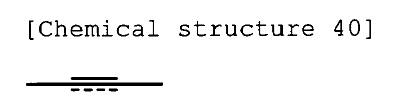 indicates 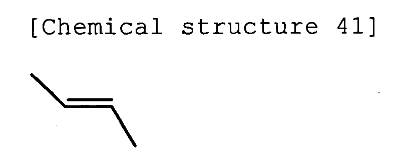 or 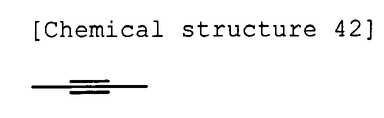 and
any one of R311, R321 or R331 is a group represented by formula (II'):

[Chemical structure 43] -X-OTs (II')

(wherein X has the above-mentioned meaning), and
remaining two of R311, R321 or R331 are hydrogen atoms or lower alkyl groups, or
in case that R331 is a group represented by above-mentioned formula (II'), R311, R321 and nitrogen atoms bonded thereto, together, form 3-8 membered nitrogen-containing aliphatic cyclic group,
p is 1, or in case that either of R311 or R321 is a group represented by above-mentioned formula (II'), p may be 0,
and
one or two of A1 through A5 is a nitrogen atom and the remaining three or four are CH].

Each reference character is same as that of formula (I) except that one of R111, R121 or R131 of formula (I'), one of R211, R221 or R231, or one of R311, R321 or R331 represents a group represented by formula (II').

As a group represented by formula (II'), particularly, a group selected from the group consisting of formula (III'): 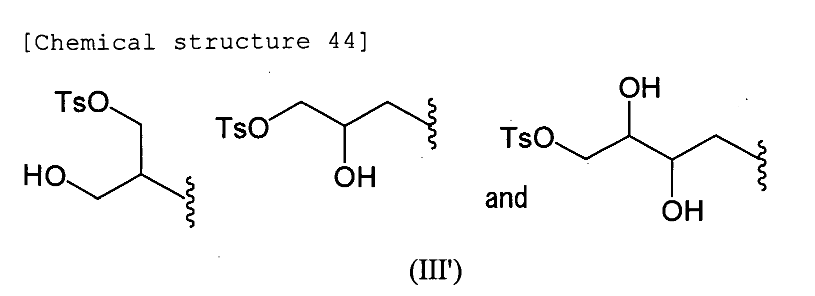 [wherein, 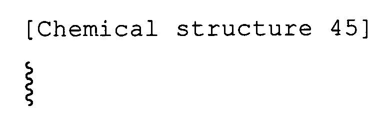 shows the bonded portion with an oxygen atom or nitrogen atom],
and of these, preferably a group represented by formula (III'-1): 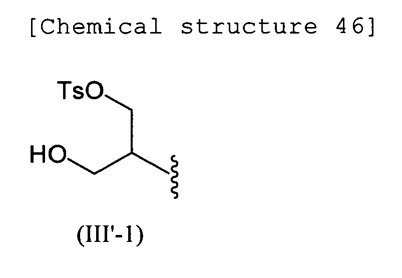 [wherein, 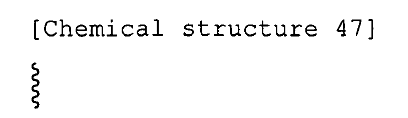 is the same as above] or a group represented by formula (III'-2): 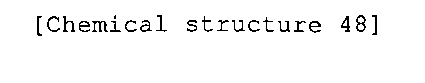 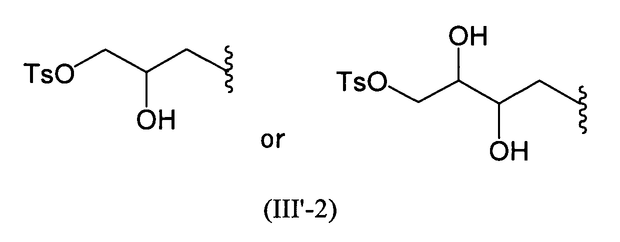 [wherein, 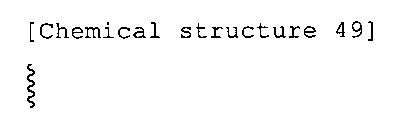 is the same as above] is exemplified.

In the diagnosis for protein conformation diseases, it is possible to employ the compounds of the present invention as the probe without labeling. For example, it is may be examined whether there is a part to be stained by contacting the compounds of the present invention with the biopsy specimen. However, the labeled compounds of the present invention generally used as the diagnostic probe for protein conformation diseases. The labels may include fluorescent material, affinity substance, enzyme substrate, and radiation nuclide. In image diagnosis for protein conformation diseases, generally the probe labeled with radiation nuclides are used. It is possible to label the compounds of the present invention with a variety of radiation nuclides by the well known method in said field. For example, ³H, ¹⁴C, ³⁵S, and ¹³¹I are conventionally used radiation nuclides, and in many cases, used in vitro. The general requirements needed for the imaging diagnostic probe and detection means thereof include a possibility of conducting image diagnosis in vivo, less damage to patients (in particular, non invasive), high detection sensitivity, and an appropriate length of half life (time to prepare the labeling probe, and diagnosis time are appropriate). Therefore, recently, positron emission tomography (PET) utilizing gamma ray having high detection sensitivity and substance transmissivity or Single Photon Emission Computed Tomography (SPECT) by use of radiation nuclides have been used. Among these, PET is preferable because it can detect 2 gamma rays that are emitted to the opposite direction from the positron emitting nuclides by a pair of detectors using the coincidence method, and provides information more excellent in resolution or quantitation. For SPECT, it is possible to label the compounds of the present invention with gamma ray emitting nuclides such as ^{99m}Tc, ¹¹¹In, ⁶⁷Ga, ²⁰¹T1, ¹²³I, and ¹³³Xe. ^{99m}Tc and ¹²³I are comparatively frequently used for SPECT. For PET, the compounds of the present invention can be labeled with positron emitting nuclides such as ¹¹C, ¹³N, ¹⁵O, ¹⁸ F, ⁶²Cu, ⁶⁸Ga, and ⁷⁶Br. Among positron emitting nuclides, ¹¹C, ¹³N, ¹⁵O, and ¹⁸F are preferable, and ¹⁸F is particularly preferable in terms of appropriateness of half life and easiness for labeling. With regard to the labeling position of radiation nuclides including positron emitting nuclides, or gamma ray emitting nuclides in the compounds of the present invention, any position is allowed, however preferable labeling position is an alkyl group and/or a phenyl ring of the compound. Such labeled compounds are also included in the present invention. For example, if the compound of the present invention is labeled with ¹⁸F, any side-chain may be labeled with ¹⁸F, or hydrogen on the ring may be substituted with ¹⁸F. For example, the hydrogen contained in any of alkyl substituent may be substituted with ¹⁸F. Although self-evident to those skilled in the art, "m" represents isomer in the metastable state.

Table 1 below shows examples of specific compounds represented by formula (I).

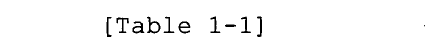

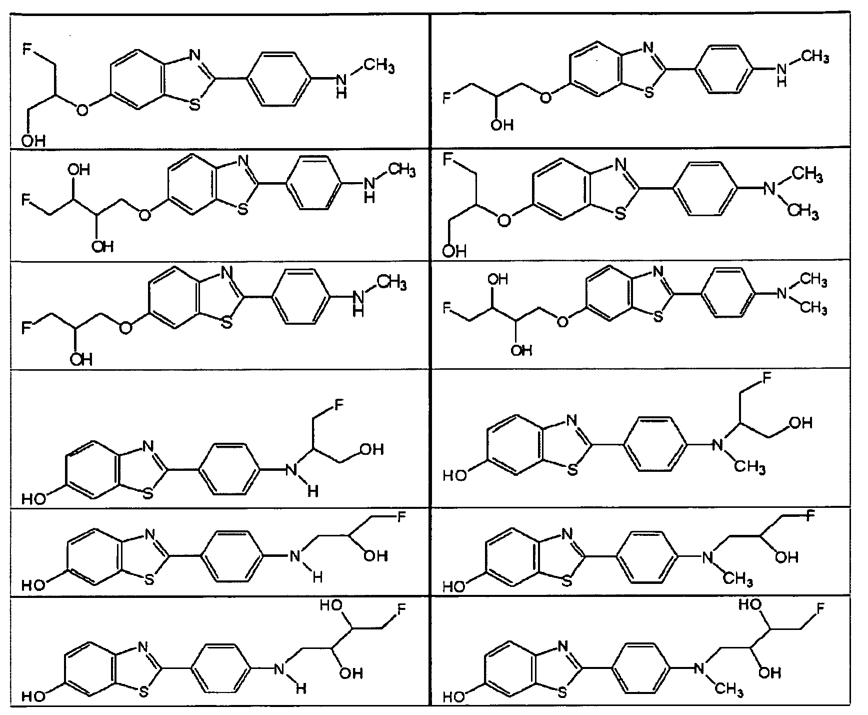

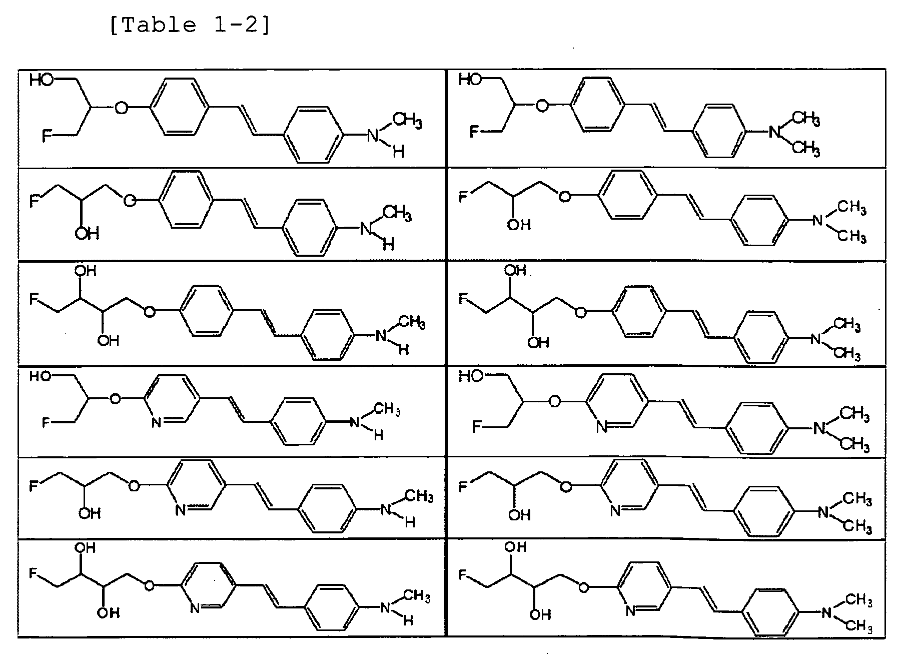

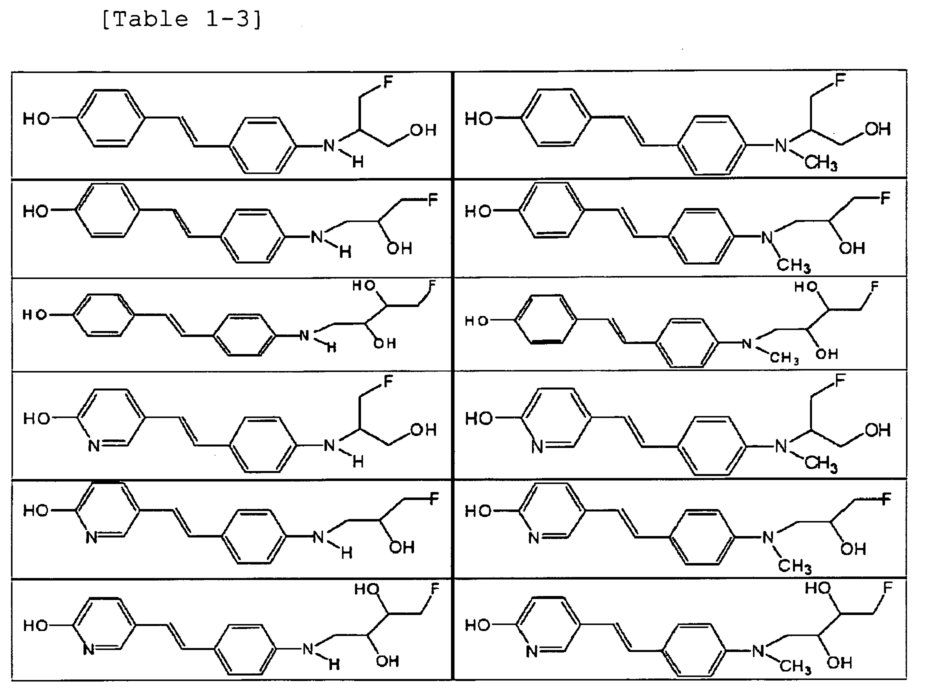

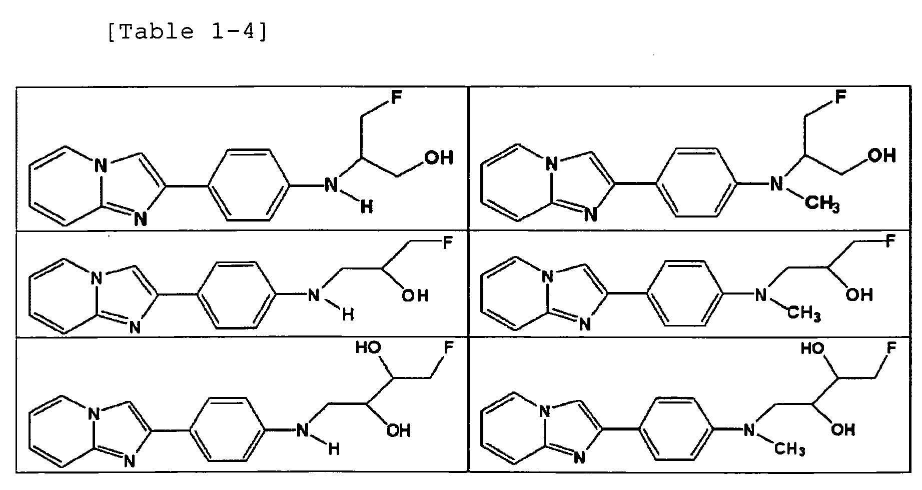

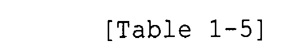

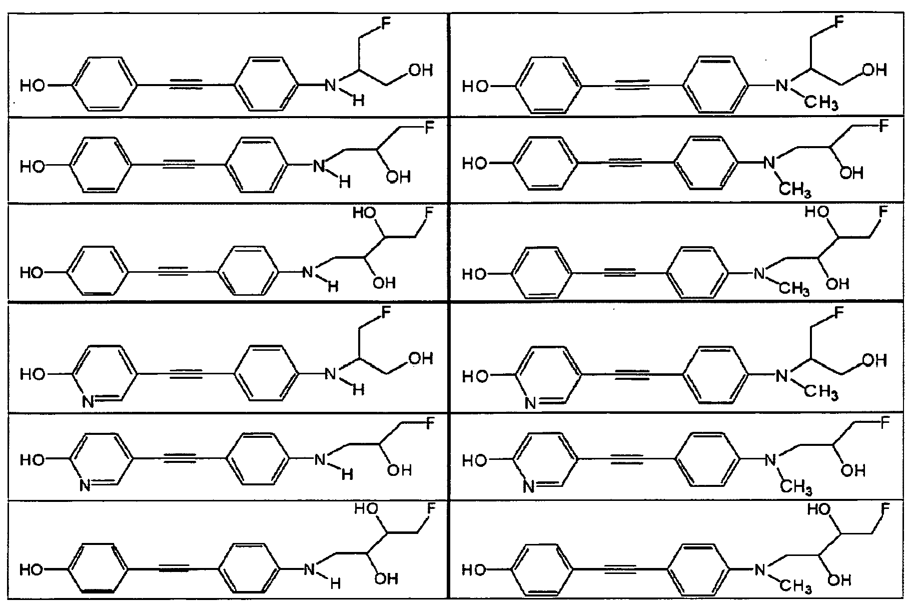

In addition, as examples of specific compounds represented by formula (I') of the present invention, compounds with TsO substituted for the fluorine atom of Table 1 above are exemplified. Here, Ts means a p-toluenesulfonyl group.

Accordingly, the invention provides the following:
(1) A compound selected from the group consisting of formula (I): 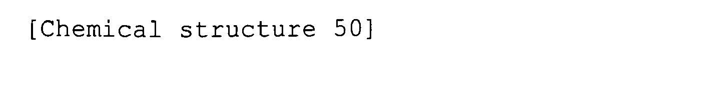 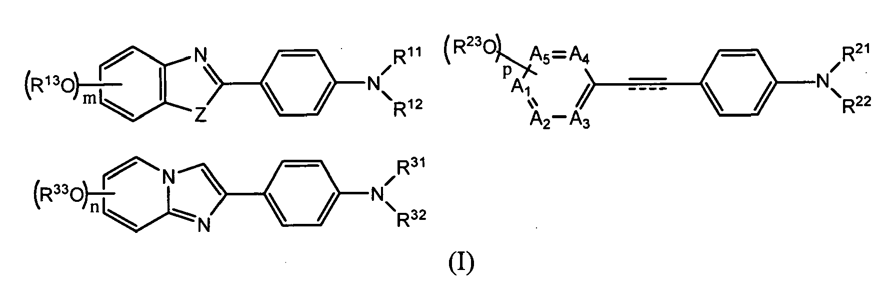 and
   [wherein any one of R11, R12, or R13 is a group represented by formula (II):

   [Chemical structure 51] -X-F (II)

   (wherein X is an alkyl group of 3 through 10 carbons substituted by 1 through 3 hydroxy groups), and
   remaining two of R11, R12 or R13 are hydrogen atoms or lower alkyl groups, or
   in case that R13 is a group represented by above-mentioned formula (II), R11, R12 and nitrogen atoms bonded thereto, together, form 3-8 membered nitrogen-containing aliphatic cyclic group,
   m is 1, or in case that either of R11 or R12 is a group represented by above-mentioned formula (II), m may be 0,
   Z represents an oxygen atom or sulfur atom;
   any one of R21, R22 or R23 is a group represented by formula (II):

   [Chemical structure 52] -X-F (II)

   (wherein X has the above-mentioned meaning), and
   remaining two of R21, R22 or R23 are hydrogen atoms or lower alkyl groups, or
   in case that R23 is a group represented by above-mentioned formula (II), R21, R22 and nitrogen atoms bonded thereto, together, form 3-8 membered nitrogen-containing aliphatic cyclic group,
   p is 1, or in case that either of R21 or R22 is a group represented by above-mentioned formula (II), p may be 0, and 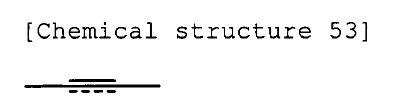 represents 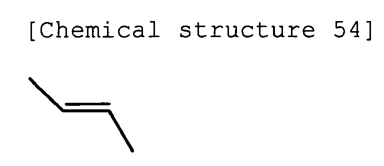 or 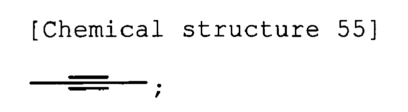 and one of R31, R32 or R33 is a group represented by formula (II):

   [Chemical structure 56] -X-F (II)

   (wherein X has the above-mentioned meaning), and
   remaining two of R31, R32 or R33 are hydrogen atoms or lower alkyl groups, or
   in case that R33 is a group represented by above-mentioned formula (II), R31, R32 and nitrogen atoms bonded thereto, together, form 3-8 membered nitrogen-containing aliphatic cyclic group,
   n is 1, or in case that either of R31 or R32 is a group represented by above-mentioned formula (II), n may be 0, and
   one or two of A1 through A5 is a nitrogen atom and the remaining three or four are CH], pharmaceutically acceptable salt or solvate thereof.
(2) The compound according to (1), pharmaceutically acceptable salt or solvate thereof, wherein X is a straight-chain or branched alkyl group of 3 through 7 carbons substituted by 1 or 2 hydroxy groups.
(3) The compound according to (1), pharmaceutically acceptable salt or solvate thereof, wherein formula (II) is a group selected from the group consisting of formula (III): 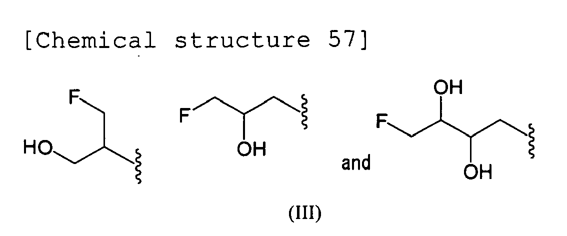 wherein, 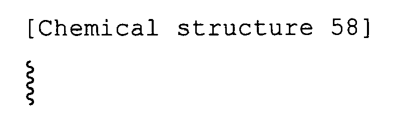 shows the bonded portion with an oxygen atom or nitrogen atom.
(4) The compound according to (1), pharmaceutically acceptable salt or solvate thereof, wherein the compound represented by formula (I) is a compound expressed by formula (I-1): 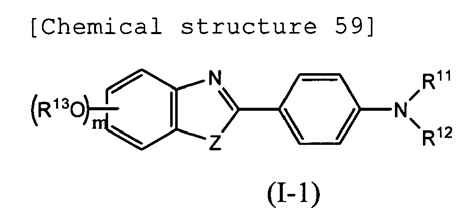 wherein any one of R11, R12, or R13 is a group represented by formula (II):

   [Chemical structure 60] -X-F (II)

   (wherein X is an alkyl group of 3 through 10 carbons substituted by 1 through 3 hydroxy groups), and is selected from the group consisting of formula (III): 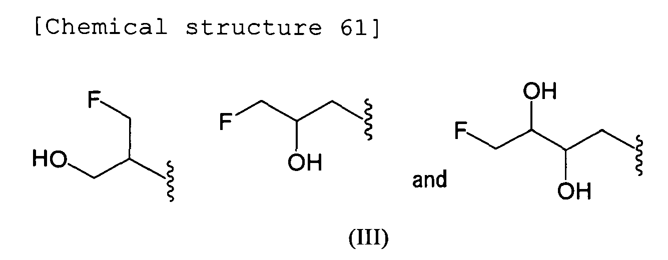 (wherein, 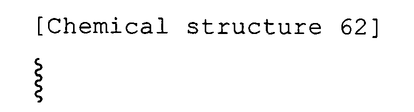 shows the bonded portion with an oxygen atom or nitrogen atom),
   and remaining two of R11, R12 or R13 are hydrogen atoms or lower alkyl groups, or
   in case that R13 is a group represented by above-mentioned formula (II), R11, R12 and nitrogen atoms bonded thereto, together, form 3-8 membered nitrogen-containing aliphatic cyclic group,
   m is 1, or in case that either of R11 or R12 is a group represented by above-mentioned formula (II), m may be 0, and
   Z represents an oxygen atom or sulfur atom.
(5) The compound according to (4), pharmaceutically acceptable salt or solvate thereof, wherein R13 is a group selected from the group consisting of formula (III): 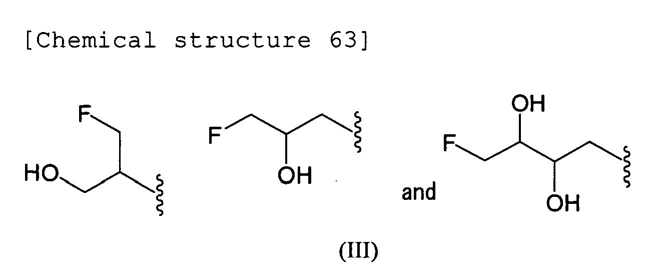 (wherein, 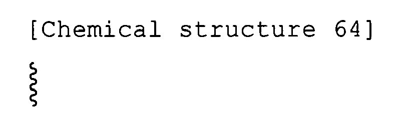 shows the bonded portion with an oxygen atom or nitrogen atom),
   and R11 and R12 are independently hydrogen atoms or low alkyl groups, or R11, R12 and nitrogen atoms bonded thereto, together, form 3-8 membered nitrogen-containing aliphatic cyclic group,
   m is 1, and
   Z is a sulfur atom.
(6) The compound according to (5), pharmaceutically acceptable salt or solvate thereof, wherein R13 is a group represented by formula (III-1): 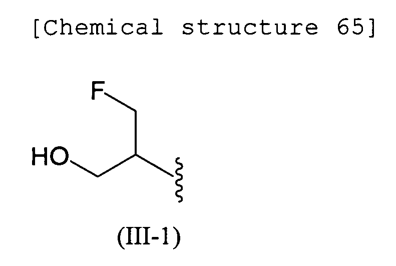 wherein, 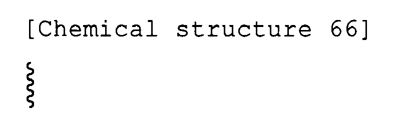 shows the bonded portion with an oxygen atom or nitrogen atom.
(7) The compound according to (1), pharmaceutically acceptable salt or solvate thereof, wherein formula (I) is a compound expressed by formula (I-2): 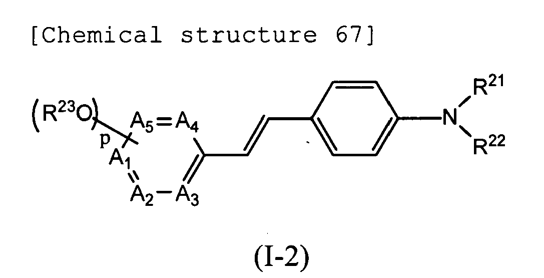 wherein any one of R22 or R23 is a group represented by formula (II):

   [Chemical structure 68] -X-F (II)

   (wherein X is an alkyl group of 3 through 10 carbons substituted by 1 through 3 hydroxy groups), and
   remaining two of R21, R22 or R23 are hydrogen atoms or lower alkyl groups, or
   in case that R23 is a group represented by formula (II):

   [Chemical structure 69] -X-F (II)

   (wherein X has the above-mentioned meaning), R21, R22 and nitrogen atoms bonded thereto, together, form 3-8 membered nitrogen-containing aliphatic cyclic group,
   p is 1, or in case that either of R21 or R22 is a group represented by above-mentioned formula (II), p may be 0, and
   one or two of A1 through A5 is a nitrogen atom and the remaining three or four are CH.
(8) The compound according to (7), pharmaceutically acceptable salt or solvate thereof, wherein R23 is a group selected from the group consisting of formula (III): 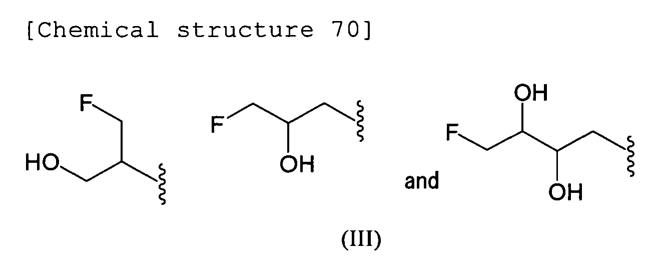 (wherein, 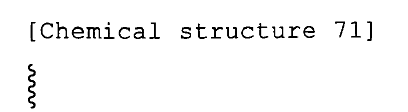 shows the bonded portion with an oxygen atom or nitrogen atom),
   and R21 and R22 are independently hydrogen atoms or low alkyl groups, or R21, R22 and nitrogen atoms bonded thereto, together, form 3-8 membered nitrogen-containing aliphatic cyclic group, and p is 1.
(9) The compound according to (8), pharmaceutically acceptable salt or solvate thereof, wherein R23 is a group represented by formula (III-1): 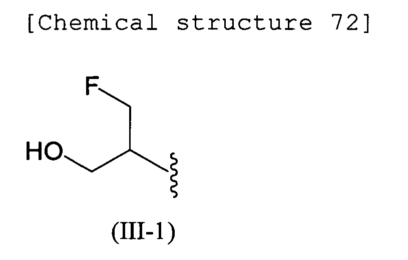 formula (III-1).
(10)The compound according to (1), pharmaceutically acceptable salt or solvate thereof, wherein the compound represented by formula (I) is either 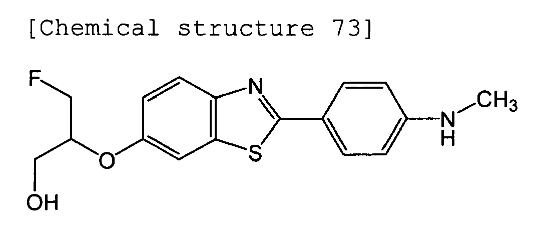 or 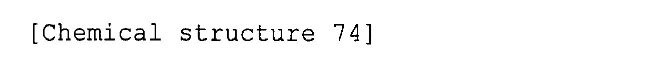 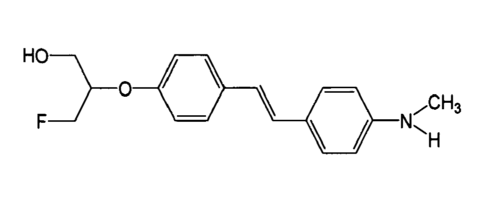
(11) The compound according to any one of (1) to (10), pharmaceutically acceptable salt or solvate thereof, which is labeled.
(12)The compound according to (11), pharmaceutically acceptable salt or solvate thereof, wherein the label is a radionuclide.
(13)The compound according to (12), pharmaceutically acceptable salt or solvate thereof, wherein the radionuclide is a gamma-ray emitting radionuclide.
(14)The compound according to (11), pharmaceutically acceptable salt or solvate thereof, wherein the label is a positron emitter.
(15)The compound according to (14), pharmaceutically acceptable salt or solvate thereof, wherein the positron emitter is selected from the group consisting of ¹¹C, 1³N, ¹⁵O, ¹⁸F, ³⁵mCl, ⁷⁶Br, ⁴⁵Ti, ⁴⁸V, ⁶⁰Cu, ⁶¹Cu, ⁶²Cu, ⁶⁶Ga, ⁸⁹Zr, ⁹⁴mTc and ¹²⁴I.
(16)The compound according to (14), pharmaceutically acceptable salt or solvate thereof, wherein the positron emitter is ¹¹C or ¹⁸F.
(17)A pharmaceutical composition comprising the compound according to any one of (1) to (16), pharmaceutically acceptable salt or solvate thereof.
(18)A pharmaceutical composition comprising the compound according to any one of (1) to (16), pharmaceutically acceptable salt or solvate thereof, and a solubilizing agent.
(19)The pharmaceutical composition according to (18), wherein the solubilizing agent is selected from the group consisting of polysolvate 80, polyethylene glycol, ethanol, and propylene glycol.
(20)The pharmaceutical composition according to any one of (17) to (19), which is an injection drug.
(21)A composition for diagnosis of conformation diseases comprising the compound according to any one of (1) to (16), pharmaceutically acceptable salt or solvate thereof.
(22)A pharmaceutical composition to treat and/or prevent conformation diseases, comprising the compound according to any one of (1) to (16), pharmaceutically acceptable salt or solvate thereof.
(23)A kit for diagnosis of conformation diseases, comprising the compound according to any one of (1) to (16), pharmaceutically acceptable salt or solvate thereof as a essential component.
(24)A composition or kit for detecting or staining proteins with beta sheet structure or neurofibrillary tangle, comprising the compound according to any one of (1) to (16), pharmaceutically acceptable salt or solvate thereof as a essential component.
(25)The kit according to (23) or (24) for diagnostic imaging.
(26)A method for treatment and/or prevention of conformation diseases in a subject, whichcomprises administering the compound according to any one of (1) to (16), pharmaceutically acceptable salt or solvate thereof to the subject.
(27)A method for diagnosis of conformation diseases in a subject, which comprises administering the compound according to any one of (1) to (16), pharmaceutically acceptable salt or solvate thereof to the subject.
(28) Use of the compound according to any one of (1) to (16), pharmaceutically acceptable salt or solvate thereof for producing a composition or kit of aid diagnosis of conformation diseases in a subject.
(29)The use of the compound according to any one of (1) to (16), pharmaceutically acceptable salt or solvate thereof for producing a pharmaceutical composition for treatment and/or prevention of conformation diseases in a subject.
(30) A method for detecting or staining proteins with beta sheet structure or neurofibrillary tangle in a sample, which comprises using the compound according to any one of (1) to (16), pharmaceutically acceptable salt or solvate thereof to stain samples.
(31) Use of the compound according to any one of (1) to (16), pharmaceutically acceptable salt or solvate thereof for producing a composition or a kit for detecting or staining proteins with beta sheet structure or neurofibrillary tangle in a sample.
(32)A compound selected from the group consisting of formula (I'): 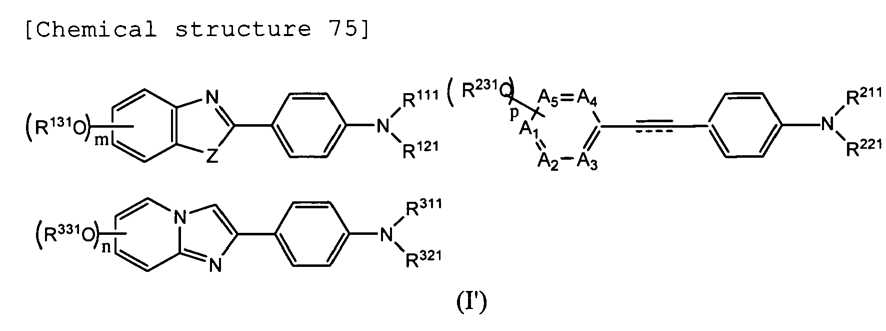 and
   wherein any one of R111, R121, or R131 is a group represented by formula (II'):

   [Chemical structure 76] -X-OTs (II')

   (wherein X is an alkyl group of 3 through 10 carbons substituted by 1 through 3 hydroxy groups), and
   remaining two of R111, R121 or R131 are hydrogen atoms or lower alkyl groups, or
   in case that R131 is a group represented by above-mentioned formula (II), R111, R121 and nitrogen atoms bonded thereto, together, 3-8 membered nitrogen-containing aliphatic cyclic group,
   m is 1, or in case that either of R111 or R121 is a group represented by above-mentioned formula (II'), m may be 0,
   Z represents an oxygen atom or sulfur atom,
   any one of R21, R22 or R23 is a group represented by formula (II') comprising:

   [Chemical structure 77] -X-OTs (II')

   (wherein X has the above-mentioned meaning), and
   remaining two of R211, R221 or R231 are hydrogen atoms or lower alkyl groups, or
   in case that R231 is a group represented by above-mentioned formula (II'), R211, R221 and nitrogen atoms bonded thereto, together, form 3-8 membered nitrogen-containing aliphatic cyclic group,
   n is 1, or in case that either of R211 or R221 is a group represented by above-mentioned formula (II'), n may be 0,
   and 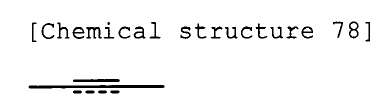 represents 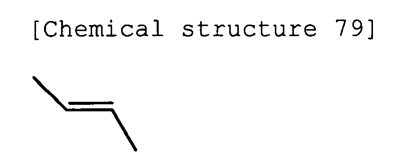 or 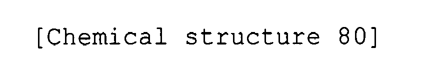 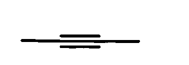 and any one of R311, R321 or R331 is a group represented by formula (II'):

   [Chemical structure 81] -X-OTs (II')

   (wherein X has the above-mentioned meaning), and
   remaining two of R311, R321 or R331 are hydrogen atoms or lower alkyl groups, or
   in case that R331 is a group represented by above-mentioned formula (II'), R311, R321 and nitrogen atoms bonded thereto, together, form 3-8 membered nitrogen-containing aliphatic cyclic group,
   p is 1, or in case that either of R31 or R32 is a group represented by above-mentioned formula (II'), p may be 0,
   and
   one or two of A1 through A5 is a nitrogen atom and the remaining three or four are CH.
(33)The compound according to (32), wherein the group represented by formula (II') is selected from the group consisting of formula (III'): 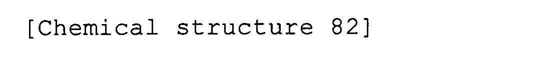 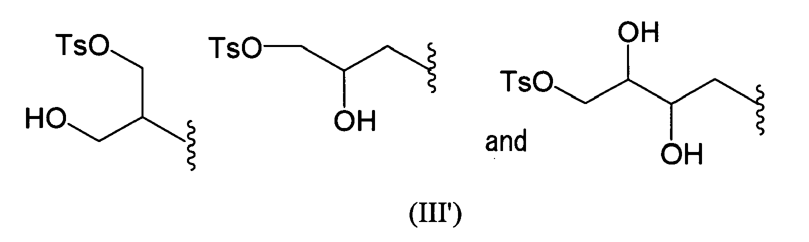 wherein, 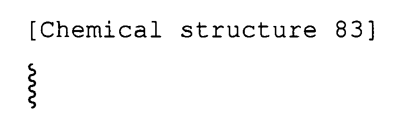 shows the bonded portion with an oxygen atom or nitrogen atom.
(34)The compound according to (32), wherein the compound represented by formula (I') is either 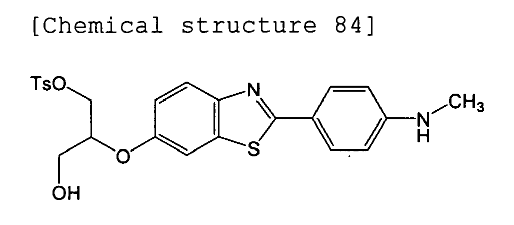 or

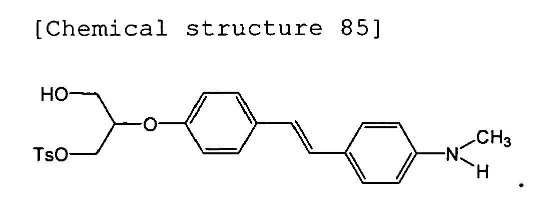

Next discussion will be made on the method for producing a compound of the present invention. The compound represented by formula (I) can be produced, for example, by the following method. 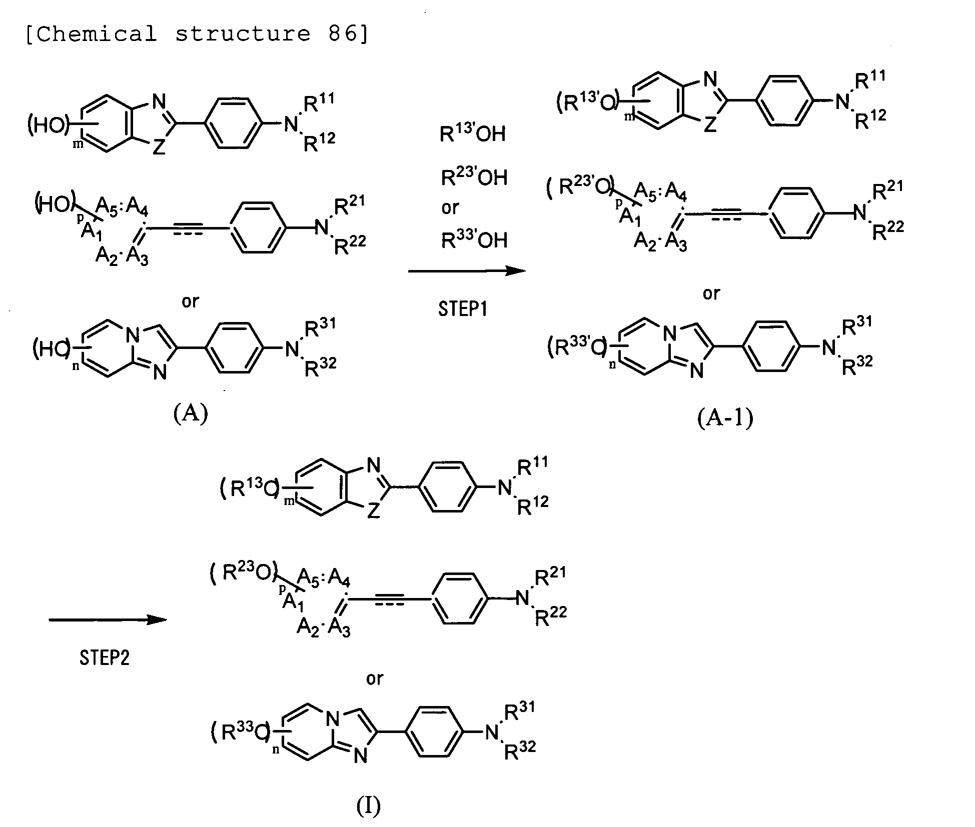 [In the scheme, R13', R23', or R33' represents a protective group of hydroxyl group which R13, R23 or R33 possesses, and other variables have the above-mentioned meanings.]

### (Step 1)

The present process is a method for producing compound (A-1) by allowing compound (A) to react with compounds R13'OH, R23'OH or R33'OH. Reactions in this process are so-called Mitsunobu reactions, and can be conducted by the method stipulated in the document (for example, "The use of diethylazodicarboxylate and triphenylphosphine in synthesis and transformation of natural products" by O. Mitsunobu, Synthesis, Volume 1, 1981, pl-28), methods that conform to this, or combining these with routine procedures in the presence of phosphine compound and azo compound.

For compound (A) used, a known compound may be used or with a known compound used as material, a compound produced by a method routinely used in the organic chemistry field or a method conforming to this may be used.

The amount of compound R13'OH, R23'OH or R33'OH used is normally between 0.5 and 10 equivalent weight per one equivalent weight of compound (A), and preferably between 1 and 5 equivalent weight.

For a phosphine compound used in the present process, for example, triphenyl phosphine, triethyl phosphine, etc. can be mentioned.

The amount of the phosphine compound used is normally between 1 and 10 equivalent weight per one equivalent weight of compound (A), and preferably between 1 and 5 equivalent weight.

For an azo compound used in the present process, for example, diethylazodicalboxylate, diisopropylazodicarboxylate, etc. can be mentioned.

The amount of the azo compound used is normally between 0.5 and 10 equivalent weight per one equivalent weight of compound (A), and preferably between 1 and 5 equivalent weight.

The reaction time is normally between 1 and 48 hours, and preferably between 1 and 24 hours.

The reaction temperature is normally from 0°C to the reflux temperature of the reaction solvent, and preferably from 10°C to 50°C.

There is no particular restriction to reaction solvents used in the present process if the solvents do not cause any hindrance to reactions, but tetrahydrofuran (THF), toluene, etc. may be mentioned.

The compound (A-1) this obtained is isolated and separated by a known separation and refinement means, such as, concentration, vacuum-concentration, crystallization, extraction by solvent, reprecipitation, chromatography, etc. or submitted to the subsequent process without conducting isolation and separation.

### (Step 2)

This process is a method for producing compound (I) of the present invention by removing the protective group of hydroxyl group which R13', R23', or R33' possesses. The protective group of hydroxyl group in the present process can be removed by methods stipulated in documents (for example, "Protective Groups in Organic Synthesis" written by T. W. Green, Version 2, John Wiley & Sons, 1991, etc.), and methods that conform to them or by combining these with routine procedures.

The compound (I) thus obtained is isolated and separated by a known separation and refinement means, such as, concentration, vacuum-concentration, crystallization, extraction by solvent, reprecipitation, chromatography, etc.

By the way, a protective group of an amino group may be introduced, as necessary, into R11, R12, R21, R22, R31, or R32 of formula (A), (A-1) or (I) and then, the protective group of the amino group may be removed.
In addition, a group represented by 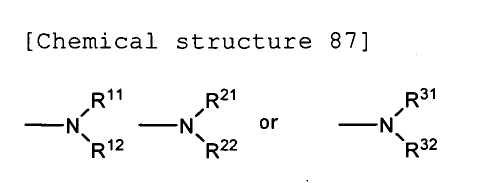 [wherein, each variable has the above-mentioned meaning] or a group with a protective group introduced in these amino groups may be derived from a nitro group.

The protective group of the amino group can be introduced by the method stipulated in "Protective Groups in Organic Synthesis" mentioned above, and methods that conform to this or by combining these with routine procedures.

In addition, the compound (I') of the present invention can be produced, for example, by the following method: 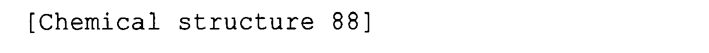 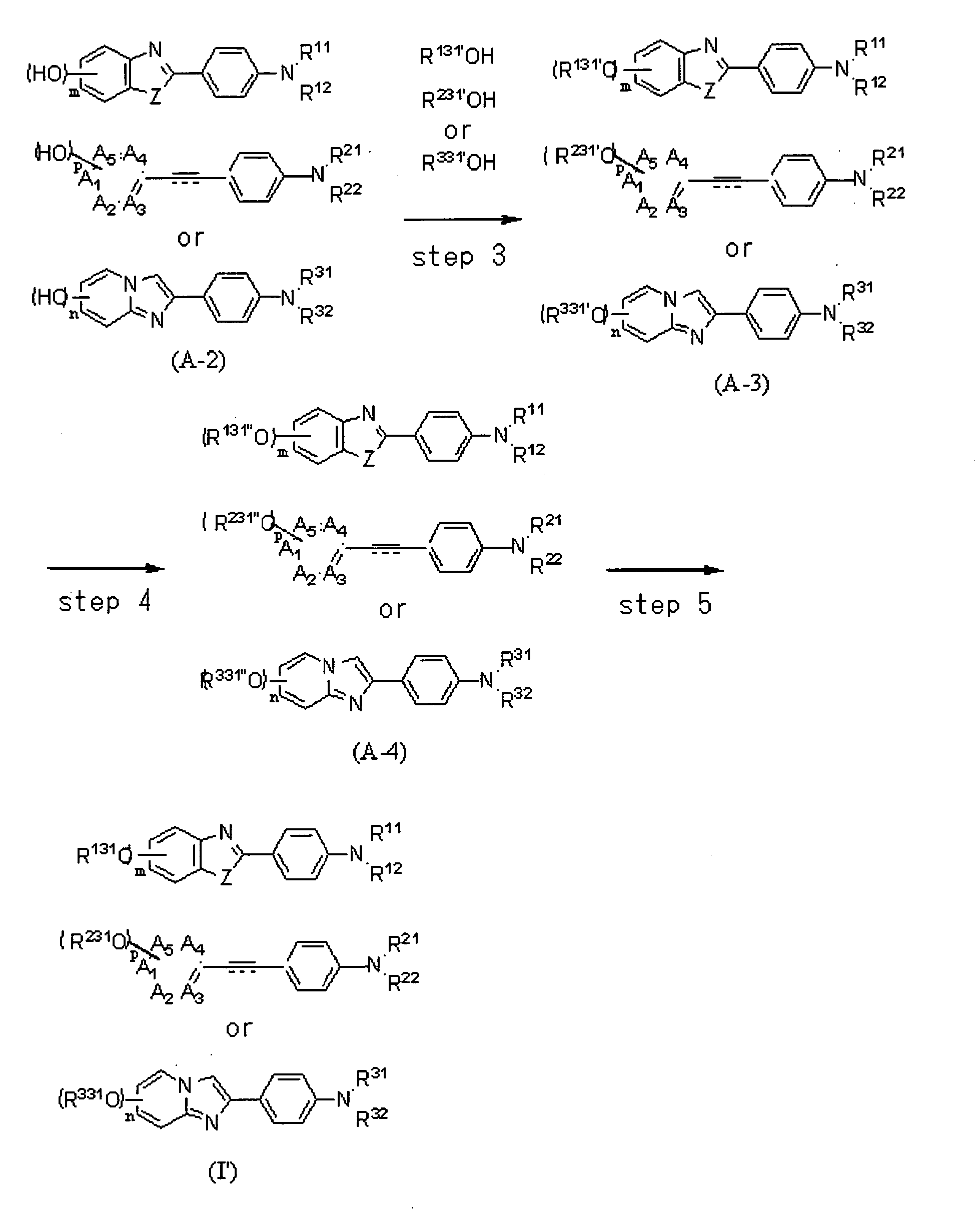 [In the scheme, each R131', R231', or R331' indicates a group represented by formula (II'-1), respectively:

[Chemical structure 89] -X'-OPro (II'-1)

(wherein, X' represents X with the hydroxyl group which the
X possesses protected and Pro represents the protective group of the hydroxyl group), and each R131", R231", or R331" indicates a group represented by formula (II'-2):

[Chemical structure 90] -X-OH (II'-2)

(wherein, X has the above-mentioned meaning)].

### (Step 3)

The present process is a method for producing compound (A-3) by allowing compound (A-2) to react with compounds R131'OH, R231'OH or R331'OH. Reactions in this process are so-called Mitsunobu reactions, and can be conducted by the method same as process 1 mentioned above, methods that conform to this, or combining these with routine procedures.

The compound (A-3) thus obtained is isolated and separated by a known separation and refinement means, such as, concentration, vacuum-concentration, crystallization, extraction by solvent, reprecipitation, chromatography, etc., or submitted to the subsequent process without conducting isolation and separation.

### (Step 4)

This process is a method for producing compound (A-4) by removing the protective group which compound (A-3) possesses.
The protective group of hydroxyl group can be removed by methods stipulated in "Protective Groups in Organic Synthesis" mentioned above, and methods that conform to this or by combining these with routine procedures.

The compound (A-4) thus obtained is isolated and separated by a known separation and refinement means, such as, concentration, vacuum-concentration, crystallization, extraction by solvent, reprecipitation, chromatography, etc., or submitted to the subsequent process without conducting isolation and refinement.

### (Step 5)

This process is a method for producing compound (I') of the present invention by allowing compound (A-4) to react with p-toluenesulfonic acid anhydride in the presence of a base.

Examples of bases used include triethylamine, diisopropylethylamine, etc.
In addition, dimethylaminopyridine, etc. may be added to the reaction system in the catalyst amount.

The amount of a base used is normally between 1 and 10 equivalent weight for 1 equivalent weight of compound (A-4) and preferably between 1 and 5 equivalent weight.

The amount of p-toluensulfonic acid used is normally between 0.5 and 1 equivalent weight for 1 equivalent weight of compound (A-4).

The reaction temperature is normally between room temperature and boiling point of solvent and preferably between 50°C and 80°C.

The reaction time is normally between 1 and 48 hours, and preferably between 1 and 24 hours.

There is no particular restriction to reaction solvents if the solvents do not cause any hindrance to reactions, but dimethoxyethane, dimethylformamide, etc. may be mentioned.

The compound (I') thus obtained is isolated and separated by a known separation and refinement means, such as, concentration, vacuum-concentration, crystallization, extraction by solvent, reprecipitation, chromatography, etc. or submitted to the subsequent process without conducting isolation and separation.

A protective group of the amino group may be, as necessary, introduced to R11, R12, R21, R22, R31 or R32 of formula (A-2), (A-3), (A-4) or (I'), then the protective group of the amino group may be appropriately removed.
In addition, a group represented by 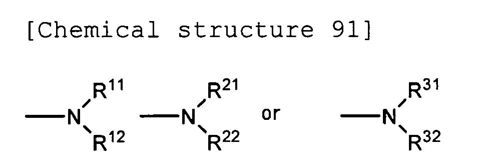 [wherein, each variable has the above-mentioned meaning] or a group with a protective group introduced into these amino groups may be derived from a nitro group.

Salts of the compounds of the present invention are also included in the present invention. Said salts can be produced by the conventional procedure using the compounds represented by the formula (I) provided by the present invention.

Specifically, if the compounds represented by the above formula (I) possess, for example, a basic group derived from an amino group, a pyridyl group and the like within said molecule, it is possible to transform said compound into a corresponding salt by processing it with an acid.

Said acid addition salts include, for example, halogenated hydroacid salts such as hydrochloride, hydrofluoric acid salt, hydrobromic acid salt, hydriodic acid salt; inorganic acid salts such as nitrate, perchlorate, sulfate, phosphate, carbonate; lower alkyl sulfonic acid salts such as methansulfonic acid salt, trifluoromethansulfonic acid salt, ethansulfonic acid salt; aryl sulfonic acid salts such as benzenesulfonic acid salt, p-toluensulfonic acid salt; organic acid salts such as fumarate, succinate, citrate, tartrate, oxalate, maleate; and organic acid salts of amino acid such as glutamate, and aspartate.

If the compounds of the present invention possesses an acidic group, for example, a carboxyl group or the like in said group, it is possible to convert said compound into a pharmaceutically acceptable corresponding salt by processing said compound with base. Said base addition salts include, for example, alkali metal salts such as sodium, potassium; alkali-earth metal salts such as calcium, magnesium; and salts by organic base such as, ammonium salt guanidine, triethylamine, and dicyclohexylamine.

Furthermore, compounds of the present invention may exist as any hydrate or solvate of free compounds or salts thereof.

In general, the nuclides may be produced by devices that are referred to as cyclotron or generator. Those skilled in the art could select the production method and device suitable to the nuclide to be produced. With the nuclide thus produced, the compounds of the invention may be labeled.

Methods for producing compounds labeled with such radionuclides are well known in this technical field. Typical methods are a chemical synthesis method, an isotope exchanging method and a biosynthesis method. The chemical synthesis method has been used widely from the past, and this does not substantially differ from an ordinary chemical synthesis method except that a radioactive starting substance is used in the former. According to this method, various nuclides are introduced into compounds. The isotope exchanging method comprises transferring ³H, ³⁵S or ¹²⁵I in a simple-structured compound into a compound having a complicated structure, thereby giving a compound having a complicated structure and labeled with the nuclide. The biosynthesis method comprises giving a ¹⁴C or ³⁵S-labeled compound to cells such as those of microorganisms, thereby producing a metabolite that has the nuclide.

Regarding the labeling position, the synthetic scheme may be planned in accordance with the object, like in ordinary synthesis, whereby the labeling substance may be introduced into the desired position. The synthetic scheme can be appropriately planned by those skilled in the art.

On the other hand, for example, when a positron emitter having a relatively short half life, such as ¹¹C, ¹³N, ¹⁵O or ¹⁸F is used, then a desired nuclide may be obtained in a (ultra)small-size cyclotron installed in an institution such as hospital, thereafter a desired compound may be labeled with it at the desired position thereof according to the above-mentioned method, and it may be directly used for diagnosis, examination or treatment, etc.

According to the methods known to those skilled in the art, the compounds of the invention may be labeled with a desired nuclide by introducing the nuclide to them at the desired position thereof.

The labeled compound of the invention may be administered to a subject either locally or systemically. The administration route includes subcutaneous, intraabdominal, intravenous, intra-arterial or intraspinal injection or infusion; and it may be selected depending on the factors such as the type of the disease, the nuclide used, the compound used, the condition of the subject and the examination site. After the probe of the invention has been administered and after a sufficient period of time has passed for binding the compound to amyloid β protein and disintegrating it, the examination site may be inspected according to means of PET or SPECT. These means may be suitably selected depending on the factors such as the type of the disease, the nuclide used, the compound used, the condition of the subject and the examination site.

The dose of the radionuclide-labeled compound of the invention varies depending on the type of the disease, the nuclide used, the compound used, the age of the subject, the physical condition, the sex, the degree of the disease and the examination site thereof. In particular, special attention should be paid to the dose equivalent to be applied to the subject. For example, the radioactivity of the compound of the invention labeled with a positron emitter such as ¹¹C, ¹³N, ¹⁵O or ¹⁸F is within a scope of generally from 3.7 megabecquerels to 3.7 gigabecquerels, preferably from 18 megabecquerels to 740 megabecquerels.

The compounds, pharmaceutically acceptable salts or solvates thereof according to the present invention are suited for treatment methods of a conformation disease later discussed, its diagnostic method, compositions for treatment, compositions for diagnosis, diagnostic kits, and the use for producing these compositions and kits as well as other uses.
In addition, the compounds of the present invention having any one of R11, R12 or R13, any one of R21, R22, or R23, or any one of R31, R32, or R33 in formula (I) selected from the group consisting of formula (III): comprising: 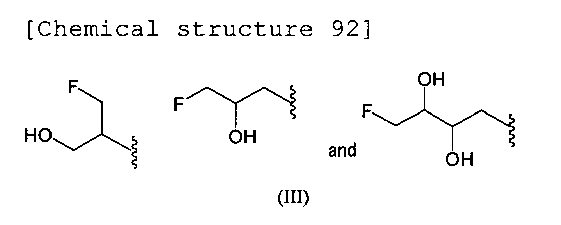 [wherein, 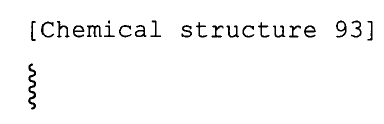 shows the bonded portion with an oxygen atom or nitrogen atom] provide extremely low or scarcely free of bone accumulation, and are suited for administration to human bodies.

The invention provides a composition for imaging diagnosis of conformation disease, which comprises the compound of the invention. The composition of the invention comprises the compound of the invention and a pharmaceutically acceptable carrier. Preferably, the compound of the invention in the composition is labeled. There are known various labeling methods as mentioned above, but for in-vivo imaging diagnosis application, the compound is preferably labeled with a radionuclide (especially for PET, positron emitter such as ¹¹C, ¹³N, ¹⁵O, ¹⁸F). Regarding its form, the composition of the invention is preferably an injectable or infusible one in view of its object. Accordingly, the pharmaceutically acceptable carrier is preferably liquid, for example, a water-based solvent such as potassium phosphate buffer, physiological saline water, Ringer solution, distilled water, or a non-aqueous solvent such as polyethylene glycol, vegetable oil and fat, ethanol, glycerin, dimethyl sulfoxide, propylene glycol, to which, however, the invention should not be limited. The blend ratio of the carrier and the compound of the invention may be suitably determined depending on the application site and the detection means, and in general, it may be from 100,000/1 to 2/1, preferably from 10,000/1 to 10/1. The composition of the invention may contain any known microbicide (e.g., antibiotic), local anesthetic (e.g., procaine hydrochloride, dibucaine hydrochloride), buffer (e.g., tris-HCl buffer, Hepes buffer), osmoregulator (e.g., glucose, sorbitol, sodium chloride).
The invention further provides a kit for imaging diagnosis of conformation disease, which comprises the compound of the invention as the essential constitutive ingredient thereof. In general, the kit comprises the components such as a compound of the invention, a solvent for dissolving it, a buffer, an osmoregulator, a microbicide and a local anesthetic, which are individually packaged or are partly combined and packaged together, and are packed in one container. The compound of the invention may be unlabeled or labeled. When the compound is unlabeled, it may be labeled before use according to the ordinary method described in the above. If desired, the compound of the invention may be provided as a solid such as a freeze-dried powder; or it may be provided as a solution prepared by dissolving it in a suitable solvent. The solvent may be the same as that for the carrier for the composition of the invention mentioned hereinabove. The other components such as buffer, osmoregulator, microbicide and local anesthetic may also be the same as those for use in the composition of the invention mentioned in the above. Various types of containers may be suitably selected and used. The containers may have a shape suitable for labeling of the compounds of the invention, or may be formed of a light-shielding material depending on the properties of the compounds. For example, the containers may have a shape of vials or syringes capable of facilitating administration to patients. The kit may comprise appliances necessary for diagnosis, such as syringe, fusion set, as well as appliances for use in PET or SPECT apparatus. In general, an instruction is attached to the kit.

Since the compounds of the invention may specifically bind to amyloid β protein, they may be used for detection and quantification of amyloid β protein in a sample by makig the compound *in-vitro* contacted with a sample, while unlabeled or after labeled. For example, the compounds of the invention may be used for amyloid β protein staining of a sample in microscopy, for colorimetry of amyloid β protein in a sample, or for quantification of amyloid β protein with a scinitillation counter. Preparing the sample for microscopy and staining it with the compound of the invention may be attained by any ordinary method known to those skilled in the art.

As so mentioned hereinabove, the compounds of the invention have high specificity to amyloid β protein. Accordingy, the compounds of the invention are useful, for example, for studies of amyloid β protein deposition-related disorders or for diagnosis thereof while alive or after death. For example, it is thought that the compounds are useful as a staining agent for seline plaque in the brain of an Alzheimer disease patient. Staining a sample, such as a brain section with the compound of the invention may be attained by any ordinary method known to those skilled in the art.

As described above, compounds of the present invention which particularly have a group selected from the group consisting of formula (III) as a terminal group: comprising: 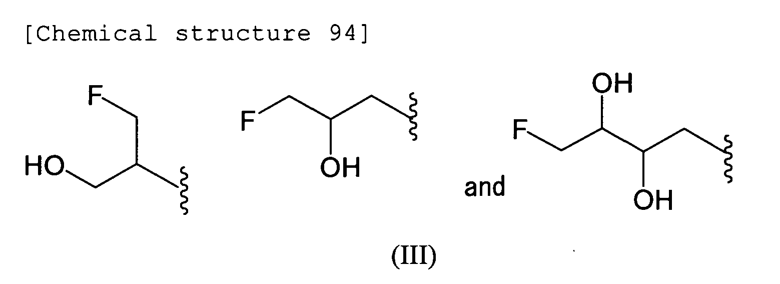 [wherein, 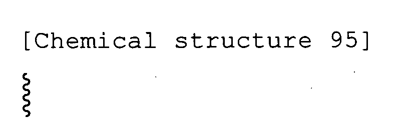 is the same as above] provide extremely low or scarcely free of bone accumulation.
Therefore, these compounds of the present invention are not only extremely safe conformation disease diagnostic probe but also achieve high safety even when they are used for therapeutic agents or preventive medicines as described later.

Accordingly, the invention relates to a composition for staining amyloid β protein in a sample, which comprises the compound or its pharmaceutically acceptable salt or solvate of the invention, and to a kit for staining amyloid β protein in a sample, which comprises, as the indispensable constitutive component thereof, the compound or its pharmaceutically acceptable salt or solvate of the invention. Further, the invention relates to a method for staining amyloid β protein in a sample, which comprises using the compound or its pharmaceutically acceptable salt or solvate of the invention. The sample suitable to staining is a brain section.

As mentioned above, it is known that β sheet structured amyloid β protein exhibits neuron toxicity. The compounds of the invention may specifically bind to β sheet structured amyloid β protein, and therefore they may inhibit its neuron toxicity. Accordingly, the compounds of the invention may be a remedial or preventive agent for conformation disease such as Alzheimer disease, of which the cause or a part of the cause is that protein having a β sheet structure.

Accordingly, the invention provides the following:
A method for treatment and/or prevention of amyloid β protein deposition-related diseases, which comprises administering a compound of formula (I) or its salt or solvate;
A method for diagnosis of amyloid β protein deposition-related diseases, which comprises using a compound of formula (I) or its salt or solvate; and
Use of the compound of formula (I) or its salt or solvate for production of a composition or kit for treatment, prevention or diagnosis of amyloid β protein deposition-related diseases.

Not specifically defined, the form of the pharmaceutical composition is preferably a liquid preparation, more preferably that for injection. The injection may be directly injected into a brain, or the pharmaceutical composition may be administered through intravenous injection or infusion since the compound of the invention has high blood-brain barrier permeability as in Example 3. The liquid preparation may be produced in any method known in the art. For producing a solution-type preparation, for example, a compound of the invention may be dissolved in a suitable carrier, injection water, physiological saline water or Ringer solution, then sterilized through a filter, and thereafter it may be filled in suitable containers such as vials or ampoules. As the case may be, the solution preparation may be freeze-dried, and may be restored to its solution with a suitable carrier just before use. A suspension-type preparation may be produced, for example, by sterilizing a compound of the invention through exposure to ethylene oxide, and then suspending it in a sterilized liquid carrier.

If those drug compositions are used as liquid prescription, in particular, prescription for injection, benzoxazole derivative of the present invention added with solubilizing agent can be used as injectable solution.
Said solubilizing agents to be used include nonionic surfactant, cationic surfactant, amphoteric surfactant. Among these, Polysorbate 80, polyethylene glycol, ethanol, or propylene glycol are preferable, and Polysorbate 80 is more preferable.

The dose of the compound of the invention to a human subject in the above-mentioned therapeutical method, preventive method and use may vary depending on the condition of a patient, the sex, the age and the body weight thereof, but in general, the dose thereof to an adult having a body weight of 70 kg may be from 0.1 mg to 1 g a day, preferably from 1 mg to 100 mg, more preferably from 5 mg to 50 mg. After a patient is treated at the dose for a predetermined period of time, the dose may be increased or decreased depending on the therapeutical result.

The compounds or their salts or solvates of the invention may be used as a diagnostic probe for conformation disease, preferably as an imaging diagnostic probe that is labeled with a radiation emitter. Further, the compounds of the invention are effective for treatment and/or prevention of conformation disease.

Accordingly, the invention relates to the following:
A compound of the invention or salt or solvate thereof for use as an imaging diagnostic probe for conformation disease;
An imaging diagnostic composition or kit that comprises a compound of the invention or salt or solvate thereof;
A pharmaceutical composition for prevention and/or treatment of conformation disease, comprising the compound of the invention or pharmaceutically acceptable salt or solvate thereof and a pharmaceutically acceptable carrier;
A method for diagnosis of conformation disease, characterized by using a compound of the invention or pharmaceutically acceptable salt or solvate thereof;
Use of the compound of the invention or pharmaceutically acceptable salt or solvate thereof for diagnosis of conformation disease;
A method for prevention and/or treatment of conformation disease, characterized by administering a compound of the invention or pharmaceutically acceptable salt or solvate thereof to a subject;
Use of the compound of the invention or pharmaceutically acceptable salt or solvate thereof for prevention and/or treatment of conformation disease; and
Use of the compound of the invention in production of a pharmaceutical composition for prevention and/or treatment of conformation disease.

The dose of the compound of the invention to a human subject in the above-mentioned therapeutical method and preventive method may be as mentioned in the above.

Among the compounds of the present invention, certain compounds recognize the change in neurofibrils, thus they can be used as the detection probe for the change in neurofibrils or staining agent for the change in neurofibrils. Therefore, the present invention relates to the use of the compounds of the present invention, or salts or solvates thereof as the detection probe for the change in neurofibrils, in particular, as the image detection probe. The preferable compounds of the present invention as the staining agent for the change in neurofibrils include THK-837 and THK-853.

Accordingly, the invention provides the following:
A composition for detecting or staining neurofibrillary tangle, which comprises a compound of the invention or salt or solvate thereof;
A kit for detecting or staining neurofibrillary tangle, which comprises a compound of the invention or salt or solvate thereof;
A method for detecting or staining neurofibrillary tangle, which comprises using a compound of the invention or salt or solvate thereof; and
Use of the compound of the invention or salt or solvate thereof for producing a composition for detecting or staining neurofibrillary tangle.

Preparing the sample for detection and staining neurofibrillary tangle therein, and the staining may be attained by any ordinary method known to those skilled in the art.

Referring now to examples below, the present invention will be described in detail and specifically, but it should be noted that the present invention shall not be limited in any way to the examples.

### EXAMPLE 1

### Synthesis of compounds of the present invention

Synthesis examples of the compounds of the present invention will be shown as follows.
For the silica gel column chromatography of the embodiment, silica gel BW300 commercially available from Fuji Silysia Chemical Ltd. was used. For the basic silica gel column chromatography which uses amino group bonding type silica gel, Chromatorex NH-DM1020 commercially available from Fuji Silysia Chemical was used.
¹H-NMR was measured by VARIAN UNITY INOVA 500 (500 MHz), JEOL JNM-LA400 (400 MHz), and Varian Gemini 2000 (300 MHz) with tetramethylsilane used as a standard reference material, and the total δ value was measured in ppm.
In addition, the mass spectrum was measured by the atmospheric pressure chemical ionization method (APCI) by the use of LCQ-Advantage available from ThermoQuest or by the use of SSQ-7000C available from FinniganMAT.
With respect to the infrared spectrum, Paragon1000 FT-IR available from Perkin-Elmer was used and for melting point measurement, BUCHI B-545 was used.

The reference character in NMR measurement have the following meanings:
- s:: Singlet
- d:: Doublet
- dd:: Double doublet
- ddd:: Double double doublet
- t:: Triplet
- dt:: Double triplet
- q:: Quartet
- m:: Multiplet
- br:: Broad
- J:: Coupling constant
- Hz:: Hertz
- CDCl3:: Dichloroform
- DMSO-d6:: Dimethylsulfoxide-D6

### Synthesis of THK-837 (159)

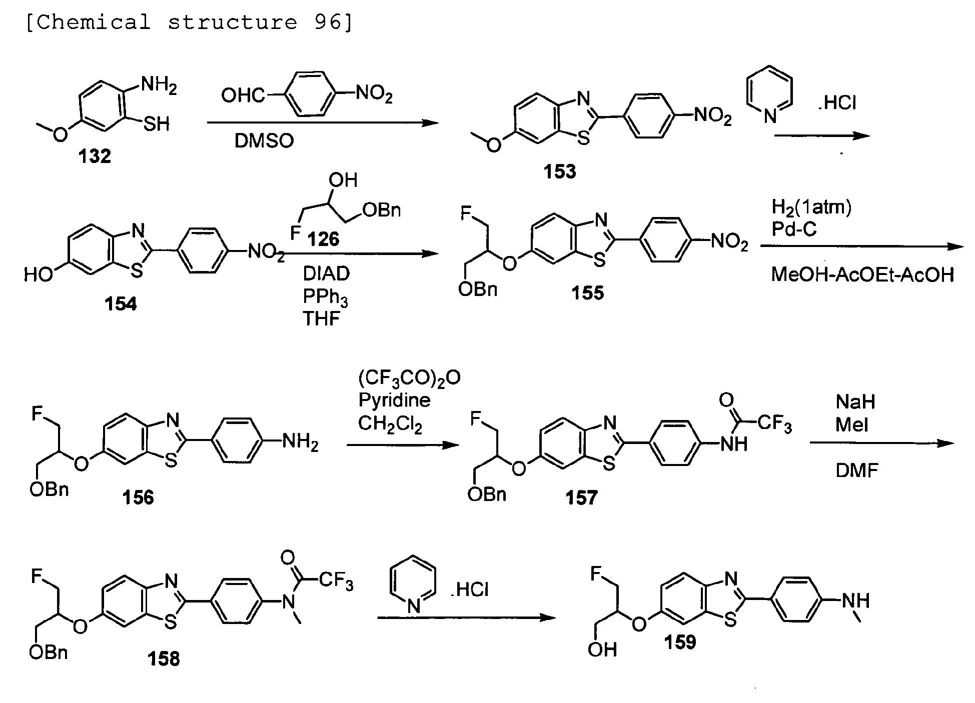

### Synthesis of 153

A solution of dimethyl sulfoxide (36 mL) of 132 (1.00g, 6.44 mmol) and 4-nitrobenzaldehyde (0.97g, 6.44 mmol) was stirred for 30 minutes at 180°C in the argon atmosphere. The reaction liquid was allowed to cool, diluted with ethyl acetate, washed with water, dried, and solvent distilled under vacuum, and the residues were recrystallized with ethyl acetate and 153 (1.57g, 85%) was obtained as orange crystal.
mp 217∼218°C, APCI-MS m/z 287[M+H]⁺

### Synthesis of 154

A mixture of 153 (1.48 g, 5.17 mmol) and pyridine hydrochloride (20 g) was stirred at 200°C for 4 hours.
The reaction liquid was allowed to cool (solidified), dissolved with water and ethyl acetate, and brought to pH1 with 10% hydrochloric acid, and after separation, the organic layer was washed with water, dried, and solvent distilled under vacuum, and 154 (1.40 g, 99%) was obtained as orange solid.
mp 262∼264°C, APCI-MS m/z 273[M+H]⁺

### Synthesis of 155

To a tetrahydrofuran (60 mL) solution of 154 (1.40 g, 5.14 mmol), 126 (1.14 g, 6.17 mmol) and triphenylphosphine (1.62 g, 6.17 mmol) were added; then, diisopropyl azodicarboxylate (1.22 mL, 6.17 mmol) was added dropwise with stirring, while ice-cooled, and stirred at room temperature for 16 hours. The reaction liquid was solvent distilled under vacuum, the residue was refined by silica gel column chromatography (elution solvent: n-hexane/ethyl acetate = 4/1) and recrystallized by ethyl acetate/n-hexane, and 155 (1.87 g, 83%) was obtained as pale yellow crystal.
mp 96∼97°C, APCI-MS m/z 439[M+H]⁺

### Synthesis of 156

To a solution of methanol/ethyl acetate/acetic acid (30/30/10 mL) of 155 (1.87g, 4.26 mmol), 10% palladium-carbon (0.38 g) was added under argon atmosphere, and stirred at atmospheric pressure, at room temperature, for 16 hours under the hydrogen atmosphere.
Palladium-carbon of the reaction liquid was filtered, the filtrate was solvent distilled under vacuum, the residue was diluted with ethyl acetate, refined by silica gel column chromatography (elution solvent: ethyl acetate/n-hexane = 3:1), and 156 (1.70 g, 98%) was obtained as pale yellow oily substance.
APCI-MS m/z 409[M+H]⁺

### Synthesis of 157

To a solution of dichloromethane (20 mL) of 156 (1.46 g, 3.57 mmol), pyridine (0.87 mL, 10.72 mmol) was added, trifuloroacetate anhydride (0.76 mL, 5.36 mmol) was added dropwise with stirring, while ice-cooled, and stirred at same temperature for 20 minutes. To the reaction liquid, iced water was added and extracted by ethyl acetate. The liquid extract was washed with water, dried, and solvent distilled under vacuum, and the residue was refined by silica gel chromatography (elution solvent: n-hexane/ethyl acetate = 3/1) and 157 (1.65 g, 92%) was obtained as pale yellow solid.
mp 121∼122°C, APCI-MS m/z 505[M+H]⁺

### Synthesis of 158

To a solution of dimethylformamide (20 mL) of 157 (1.65 g, 3.27 mmol), 60% sodium hydride (0.20 g, 4.91 mmol) was added with stirring, while ice-cooled, and stirred for 5 minutes at the same temperature, iodomethane (0.67 mL, 9.81 mmol) was added and stirred for one hour at room temperature. To the reaction liquid, saturated ammonium chloride solution was added with stirring, while ice-cooled, and extracted by ethyl acetate. The liquid extract was washed with water, dried, and solvent distilled under vacuum, and the residue was refined by silica gel chromatography (elution solvent: n-hexane/ethyl acetate = 9/1) and 158 (1.22 g, 72%) was obtained as pale yellow oily substance.
APCI-MS m/z 519[M+H]⁺

### Synthesis of 159

A mixture of 158 (1.22 g, 2.35 mmol) and pyridine hydrochloride (15 g) was stirred at 180°C for 2 hours.
The reaction liquid was allowed to cool (solidified) and dissolved with water and ethyl acetate, and after separating, the organic layer was washed with water, dried, and solvent distilled under vacuum, and 159 (0.34 g, 44%) was obtained as pale yellow crystal.
mp 162∼163□, 1H NMR(300MHz, CDCl₃)δ 1.90(1H,t,J=6.4Hz,D₂O disappeared), 2.91(3H,s), 3.90∼4.02(2H,m), 4.11(1H,s,D₂O disappeared), 4.54∼4.80(3H,m), 6.65(2H,d,J=8.3Hz), 7.11(1H,dd,J=8.8,2.0Hz), 7.45(1H,d,J=2.0Hz), 7.88 (3H,d,J=8.8Hz)
IR (Nujol) 3380,1615cm-1, APCI-MS m/z 333[M+H]+

### Synthesis of THK852

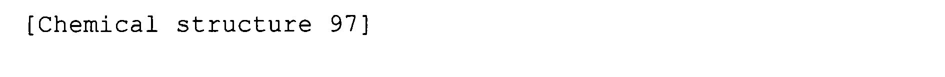

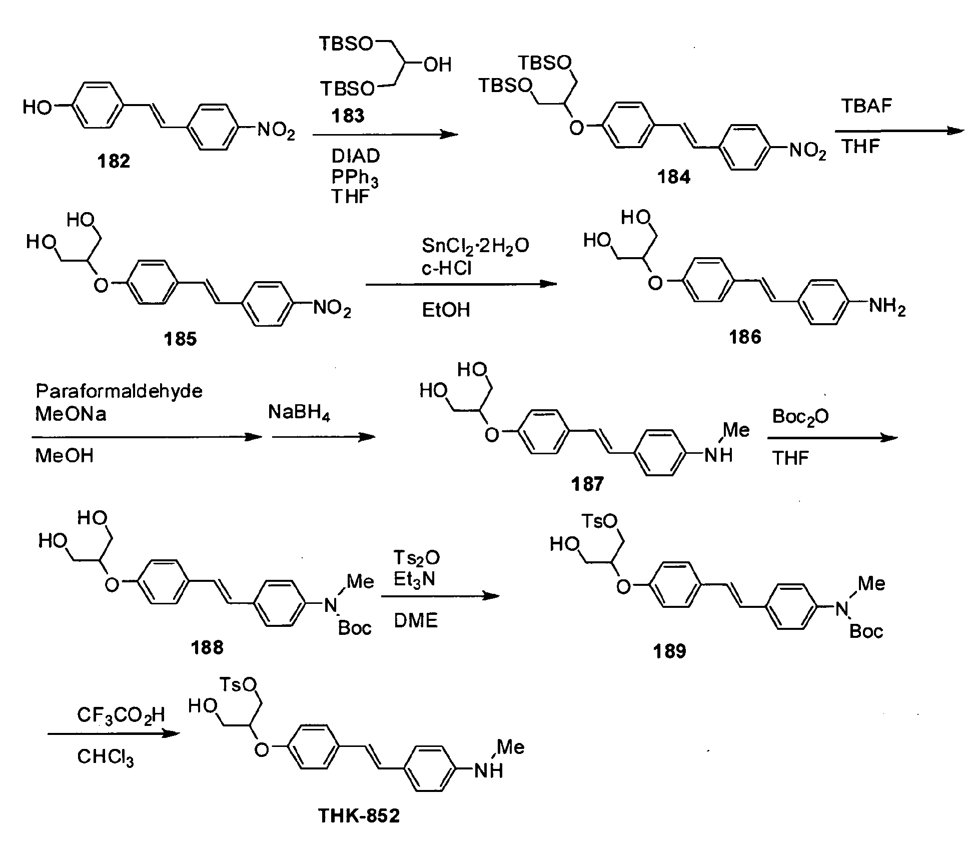

### Synthesis of 184

To a mixture of 182 (1.00 g, 4.15 mmol), 183 (1.60 g, 4.97 mmol), triphenylphosphine (1.31 g, 4.97 mmol), and tetrahydrofuran (40 mL), 126 (1.14 g, 6.17 mmol), diisopropyl azodicarboxylate (0.99 mL, 4.97 mmol) was added dropwise with stirring, while ice-cooled, under the argon atmosphere, and stirred at the same temperature for 1 hour and at room temperature for 3 days. The reaction liquid was solvent distilled under vacuum, the residue was refined by silica gel column chromatography (elution solvent: ethyl acetate /n-hexane = 1/20) and crude 184 (2.53g) was obtained as yellow oily substance. The product was not further refined but used in the subsequent process.
APCI-MS m/z544[M+H]⁺

### Synthesis of 185

To a solution of tetrahydrofuran (10 mL) of 184 (2.53 g), 1M tetrabutylammonium fluoride/tetrahydrofuran solution (8.29 mL, 8.29 mmol) was added dropwise, and stirred at room temperature for 1 hour. The reaction liquid wad diluted with water and extracted by ethyl acetate. The organic layer was washed with water, dried, and solvent distilled under vacuum, the residue was refined by silica gel column chromatography (elution solvent: ethyl acetate/n-hexane = 1/1 and ethyl acetate) and 185 (1.08 g, 82% from 182) was obtained as yellow crystal by recrystallization from ethyl acetate.
mp 129∼129.5°C, IR (Nujol)3334cm⁻¹, APCI-MS m/z316[M+H]⁺

### Synthesis of 186

A mixture of 185 (1.08 g, 3.43 mmol), ethanol (41 mL), SnCl2·2H2O (4.02 g) and concentrated hydrochloric acid (1.61 mL) was heated and refluxed for one hour. After allowing it to cool, water-ethyl acetate was added to the reaction liquid, brought to have pH8 by potassium carbonate aqueous solution, and separated. The organic layer was washed with water, dried, and solvent distilled under vacuum, the residue was refined by silica gel column chromatography (elution solvent: ethyl acetate/n-hexane = 1/1 and 2/2) and 186 (840 g, 85%) was obtained as pale yellow solid.
mp 217∼218°C, APCI-MS m/z286[M+H]⁺

### Synthesis of 187

A mixture of 186 (840 mg, 2.84 mmol), paraformaldehyde (440 mg, 14.7 mmol), sodium methoxide (28% methanol solution: 2.84 g, 14.7 mmol), and methanol (56 mL) was heated and refluxed for 2 hours under the argon atmosphere. The reaction liquid was ice-cooled, sodium boron hydride (560 mg, 14.7 mmol) was added, stirred for 5 minutes at the same temperature, and then, heated and refluxed for 1 hour. The reaction liquid is brought to room temperature, saturated ammonium chloride aqueous solution was added, water was added, and it was extracted by ethyl acetate. The liquid extract was washed with water and dried, solvent distilled under vacuum, and 187 (880 mg, quantitative) was obtained as pale yellow solid.
mp 164∼165°C, APCI-MS m/z300[M+H]⁺

### Synthesis of 188

To a solution of tetrahydrofuran (20 mL) of 187 (880 mg, 2.66 mmol), (Boc)₂O (0.73 mL, 3.19 mmol) was added, and stirred at room temperature for 3 hours, and 50°C for 14 hours. The liquid reactant was solvent distilled under pressure, the residue was washed with n-hexane and dried, and 188 (1.03 g, 96%) was obtained as a pale yellow solid.
mp142∼143°C APCI-MS m/z400[M+H]⁺
mp142∼143°C, APCI-MS m/z400[M+H]⁺

### Synthesis of 189

To a solution of ethylene glycol dimethylether (30 mL) of 188 (1.03 g, 2.58 mmol), triethylamine (0.54 mL, 3.87 mmol), toluenesulfonic acid anhydride (840 mg, 2.58 mmol) was added and heated and refluxed for 1 hour. The reaction liquid is solvent distilled under vacuum, the residue was refined by silica gel column chromatography (elution solvent: ethyl acetate/n-hexane = 1/3 to 2/1) and 189 (540 g, 37%) was obtained as pale yellow foams.
APCI-MS m/z 554[M+H]⁺

### Synthesis of THK-852

To a solution of chloroform (12 mL) of 189 (880 mg, 1.59 mmol), trifuloroacetate (8 mL) was added dropwise with stirring while ice-cooled, and stirred for 1 hour at room temperature. To the reaction liquid, iced water was added; then, diluted with ethyl acetate, and brought to be pH8 with potassium carbonate aqueous solution, and separated. The organic layer was washed with water, dried, and solvent distilled under vacuum. The residue was refined by silica gel column chromatography (elution solvent: ethyl acetate/n-hexane = 1/2) and THK-852 (436 mg, 60%) was obtained as pale yellow crystal.
mp 122∼123.5°C, ¹H NMR(400MHz, CDCl₃) δ 2.43(3H,s), 2.87(3H, s), 3.81(1H, dd, J=12, 4.9Hz), 3.89(1H, dd, J=12, 4.3Hz), 4.23∼4.29(2H, m), 4.48∼4.54(1H, m), 6.60(2H, d, J=8.6Hz), 6.81(2H,d,J=8.6Hz), 6.82(1H,d,J=16Hz), 6.90(1H,d,J=16Hz), 7.30∼7.38(6H,m), 7.76(2H,d,J=7.8Hz)
IR (Nujol)3435, 1612cm⁻¹, APCI-MS m/z454[M+H]⁺

### Synthesis of THK-853

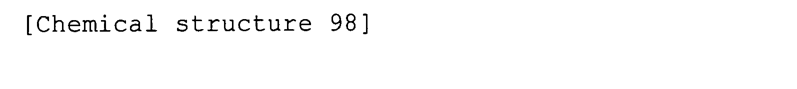

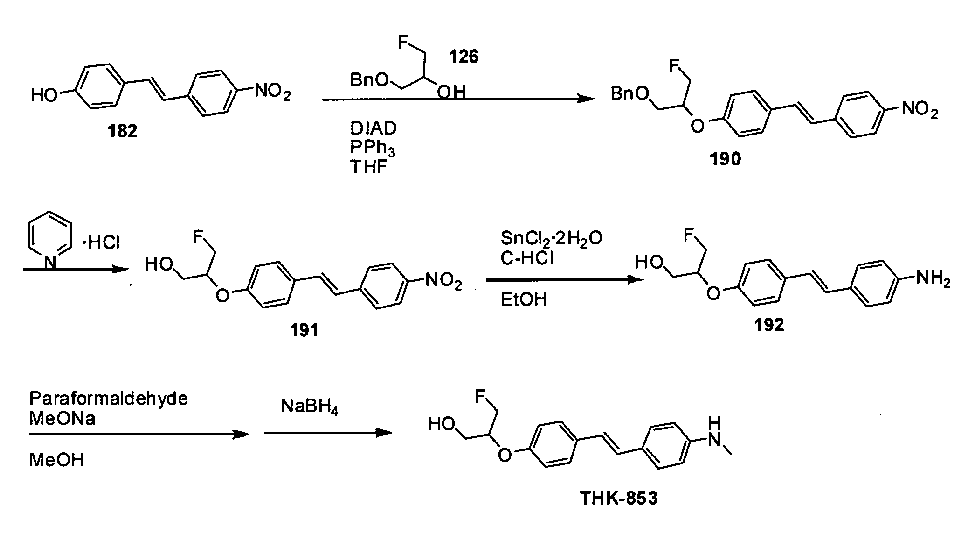

### Synthesis of 190

To the mixture of 182 (1.00g, 4.15mmol), 126 (0.92g, 4.97mmol), triphenylphosphine (1.31g, 4.97mmol), and tetrahydrofuran (40ml),diisopropyl azodicarboxylate (0.99ml, 4.97mmol) was, being cooled with ice and stirred in an anrgon atmosphere, added dropwise, and was stirred at the same temperature for 1 hour and at the room temperature for 3 days. The solvent of the reaction solution was distilled off under reduced pressure. The residue was refined with silica gel column chromatography (elution solvent: ethyl acetate/n-hexane=1/4) to obtain 190 (1.78g, quantitative) as yellow oily substance.
APCI-MS m/z408[M+H]⁺

### Synthesis of 191

The mixture of 190 (1.60 g, 3.93 mmol), and pyridine hydrochlorid (10g) was stirred at 180°C for 40 minutes. The reaction solution was cooled, and dissolved with water and ethyl acetate. It was adjusted to have pH 1 with 10% hydrochloric acid, and separated. The organic layers were washed with water, dried, and the solvent was distilled off under reduced pressure. The residue was refined with silica gel column chromatography (elution solvent: ethyl acetate/n-hexanee=1/4 to 1/1) to obtain 191 (1.03 g, 82%) as yellow solid.
mp113∼114°C, APCI-MS m/z318[M+H]⁺

### Synthesis of 192

The mixture of 191 (1.03 g, 3.25 mmol), ethanol (40 ml), SnCl₂/2H₂O (3.81 g), and concentrated hydrochloric acid (1.53 ml) was heated and refluxed for 1.5 hours. After cooled down, to the reaction solution, water-ethyl acetate was added. It was adjusted to have pH 8 with potassium carbonate aqueous solution, and separated. The organic layers were washed with water, dried, and the solvent was distilled off under reduced pressure. The residue was recrystallized from ethyl acetate to obtain 192 (820 mg, 87%) as the light yellow crystal.
mp 194∼195°C, APCI-MS m/z288[M+H]⁺

### Synthesis of THK-853

The mixture of 192 (820 mg, 2.85 mmol), paraformaldehyde (430 mg, 14.2 mmol), sodium methoxide (28% methanol solution: 2.74 g, 14.2 mmol), and methanol (55 ml) was heated and refluxed for 2 hours in an argon atmosphere. The reaction solution was cooled with ice, and sodium borohydride (540 mg, 14.2 mmol) was added. After being stirred at the same temperature for 5 minutes, it was heated and refluxed for 1 hour. The reaction solution was heated to room temperature, the saturated ammonium chloride aqueous solution and water were added, and extracted with ethyl acetate. The extracted solution was washed with water and dried, and the solvent was distilled off under reduced pressure. The residue was refined with silica gel column chromatography (elution solvent: ethyl acetate/n-hexanee=1/1) to obtain THK-853 (692 mg, 80%) as colorless crystals.
mp 156∼156.5°C,
¹H NMR(400MHz,CDCl₃)δ 1.91(1H,br,D₂O disappeared), 2.86(3H,s), 3.87∼3.97(2H,m), 4.51∼4.74(3H,m), 6.59(2H,d,J=8.5Hz), 6.84(1H,d,J=16Hz), 6.90∼6.97(3H,m), 7.34(2H,d,J=8.8Hz), 7.40(2H,d,J=8.8Hz) IR (Nujol)3588,3387,1605cm⁻¹, APCI-MS m/z302[M+H]⁺

### Synthesis of THK-857

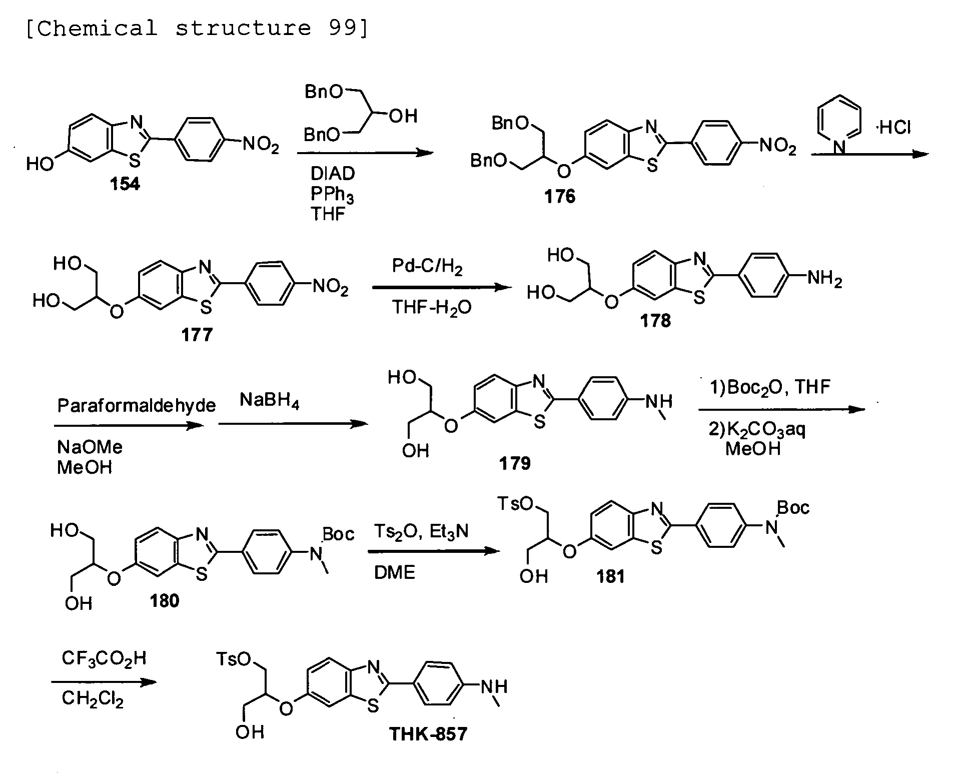

### Synthesis of 176

To the tetrahydrofuran (60ml) suspension of 154 (1.50g, 5.51mmol), 1,3-dibenzyloxy-2-propanol (1.80g, 6.61mmol) and triphenylphosphine (1.73g, 6.61mmol) were added, and diisopropyl azodicarboxylate (1.31 ml, 6.61 mmol) was, being cooled with ice and stirred in an argon atmosphere, added dropwise, and stirred at room temperature for 16 hours. 1,3-dibenzyloxy-2-propanol (0.45 g, 1.65 mmol) and triphenylphosphine (0.43 g, 1.65 mmol) were added, and diisopropyl azodicarboxylate (0.33 ml, 1.65 mmol) was, being cooled with ice and stirred, added dropwise, and stirred at room temperature for 3 days. The solvent of reaction solution was distilled off under reduced pressure, and the residue was refined with silica gel column chromatography (elution solvent: ethyl acetate/n-hexane=1/9) and recrystallized from ethyl acetate-n-hexane to obtain 176 (1.99 g, 68%) as yellow crystals.
mp 94∼95°C, APCI-MS m/z527[M+H]⁺

### Synthesis of 177

The mixture of 176 (1.99 g, 3.78 mmol) and pyridine hydrochlorid (20g) was heated at 180°C for 2.5 hours. The reaction solution was allowed to stand (solidified), and was dissolved with water and ethyl acetate, and 10 % hydrochlorid was added so as to make pH 1. After separating, the organic layers were washed with water, dried, and distilled off under reduced pressure to obtain 177 (1.00 g, 99%) as an orange solid.
mp 202∼203°C, APCI-MS m/z347[M+H]⁺

### Synthesis of 178

To the tetrahydrofuran/water (40/4ml) solution of 177 (1.00 g, 2.89 mmol), 10% palladium-carbon (moisture about 50%: 400 mg) was added in an argon atmosphere, and stirred at room temperature and normal pressure for 3 hours in a hydrogen atmosphere. Palladium-carbon of the reaction solution was filtrated. The filtrate was distilled off under reduced pressure, and the residue was refined with silica gel column chromatography (elution solvent: ethyl acetate/n-hexane=1/1 to ethyl acetate) to obtain 178 (0.90 g, 98%) as a yellow solid.
mp 181∼182°C, APCI-MS m/z 347[M+H]⁺

### Synthesis of 179

The mixture of 178 (900 mg, 2.85 mmol), paraformaldehyde (430 mg, 14.2 mmol), sodium methoxide (28% methanol solution: 2.74 g, 14.2 mmol), methanol (60 ml), and tetrahydrofuran (20 ml) was heated and refluxed for 2.5 hours in an argon atmosphere. The reaction solution was cooled with ice, sodium borohydride (540 mg, 14.2 mmol) was added, and heated and refluxed for 10 minutes. The reaction solution was cooled to room temperature, and the saturated ammonium chloride aqueous solution was added. After being stirred at room temperature for 10 minutes, water was added and the solution was extracted with ethyl acetate. The extracted solution was washed with water and dried, and the solvent was distilled off under reduced pressure. The residue was refined with silica gel column chromatography (elution solvent: ethyl acetate) to obtain 179 (890 mg, 94%) as an orange solid.
mp 179∼181°C, APCI-MS m/z331[M+H]⁺

### Synthesis of 180

To the tetrahydrofuran (50 ml) solution of 179 (890 mg, 2.69 mmol), (Boc)₂O (0.74 ml, 3.23 mmol) was added, and the solution was stirred at room temperature for 1 hour and was stirred while being heated and refluxed for 2 hours. Then, (Boc)₂O (3.72 ml, 16.1 mmol) was added, and the solution was heated and refluxed for 16 hours. The reaction solution was cooled to room temperature, and potassium carbonate aqueous solution and methanol were added, the solution was heated and refluxed for 2 hours. The reaction solution was cooled to room temperature, and was extracted with ethyl acetate. The extracted solution was washed with water, dried, and the solvent was distilled off under reduced pressure. The residue was refined with silica gel column chromatography (elution solvent: ethyl acetate/n-hexane=1/9 to ethyl acetate) to obtain 180 (910 mg, 78%) as a light yellow solid.
mp150∼151°C, APCI-MS m/z431[M+H]⁺

### Synthesis of 181

To the ethylene glycol dimethyl ether (40 ml) suspension of 180 (910 ml, 2.11 mmol), triethylamine (0.44ml, 3.17mmol) and toluenesulfonic anhydride (690 mg, 2.11 mmol) were added, and the solution was heated and refluxed for 1 hour. The solvent of reaction solution was distilled off under reduced pressure, and the residue was refined with silica gel column chromatography (elution solvent: ethyl acetate/n-hexane=1/2 to ethyl acetate) to obtain 181 (650 mg, 52%) as light yellow foamy solid.
APCI-MS m/z 585[M+H]⁺

### Synthesis of THK-857

To the chloroform (12 ml) solution of 181 (910 ml, 1.56 mmol), trifluoroacetic acid (8 ml) was, being cooled with ice and stirred, added dropwise, and was stirred at room temperature for 1 hour. To the reaction solution, the cold water was added, was diluted with ethyl acetate, and potassium carbonate aqueous solution was added so as to make pH 9, and the solution was separated. The organic layers were washed with water and dried, and the solvent was distilled off under reduced pressure. The residue was refined with silica gel column chromatography (elution solvent: ethyl acetate/n-hexane=1/1) and recrystallized with ethyl acetate to obtain THK-857 (508 mg, 67%) as light yellow crystals.
mp163.5∼164.5°C, ¹H NMR(500MHz, DMSO-d₆) δ 2.35(3H,s), 2.75(3H,d,J=5.0Hz), 3.54∼3.63(2H,m), 4.24(1H,dd,J=11,5.8Hz), 4.32(1H,dd,J=11,2.7Hz), 5.06(1H,t,J=5.6Hz,D₂O disappeared), 6.43(1H,q,J=5.0Hz,D₂O disappeared), 6.64(2H,d,J=8.6Hz), 6.95(1H,dd,J=8.9,2.4Hz), 7.39(2H,d,J=8.1Hz), 7.50(1H,d,J=2.4Hz), 7.74(2H,d,J=8.1Hz), 7.75(1H,d,J=8.9Hz), 7.78(2H,d,J=8.6Hz)
IR (Nujol)3387,3197,1610cm⁻¹,APCI-MS m/z485[M+H]⁺

### EXAMPLE 2

Staining test of the compounds of the persent invention on the brain section of patients with Alzheimer's disease

The procedure of chromosome test of the compounds of the present invention on the brain section of patients with Alzheimer's disease is explained as follows.
(1) The brain samples from the temporal lobe or hippocampus of patients pathologically definitely diagnosed as having Alzheimer's disease and normal elderly individuals were used. The samples were obtained from Choju Medical Institute in Fukushimura Hospital, our collaborative research institute, and the consent for the use for research purpose was obtained from the patients' deceased family members (Fukushimura Hospital, Ethic Committee Approval No. 20).
(2) The brain tissue embedded in the paraffin was thinly sectioned into 6 or 8 µm in thickness, extended on the slide glass, and dried. The paraffined brain section was deparaffined by washing with xylene 10 minutesx2, 100% ethanol 5 minutesx2, 90% ethanol 5 minutes, and running water 10 minutes in this order.
(3) As the pretreatment of staining by the compound of the present invention, the treatment to remove autofluorescence by lipofuscin was conducted. First, the deparaffined section was soaked in 0.25% KMnO₄ solution for 20 minutes. It was washed with PBS 2 minutes×twice, soaked in 0.1% K₂S₂O₅/oxalic acid solution for about 5 seconds, and further washed with PBS 2 minutes×three times.
(4) From 100µM of the compound solution of the present invention dissolved in 50% ethanol, about 150µl was added dropwise, and was reacted for 10 minutes. After being soaked in the tap water 5 times, it was encapsulated with Flour Save Reagent (Calbiochem) and examined with a fluorescence microscope (Nikon, Eclipse 80i). The images were taken with digital camera (Nikon Dxin1200F or Cool SNAP ES of Photometrics).

The results of above mentioned staining test on the compound of the present invention are shown in Figs 1 and 2. THK-837 bound to amyloid β protein in the brain section of the patients with Alzheimer's disease (Fig. 1). THK-835 bound to amyloid β protein in the brain section of the patients with Alzheimer's disease (Fig. 3). Like this, it was found that the compounds of the present invention specifically recognize amyloid β protein and the change in neurofibrils in the brain section of the patients with Alzheimer's disease.

### EXAMPLE 3

### Characteristics of the compounds of the present invention

The test methods related to the characteristics of the compounds of the present invention are explained below.

### Examination using the [¹⁸F] labeled compound

The compounds of the present invention can be labeled using the method known to those skilled in the art. Hereinafter, the synthesis examples of some [¹⁸F] labeled compounds of the present invention are shown below.

### The labeling synthesis of [¹⁸F] THK-837

¹⁸F⁻ was synthesized by radiating 12 MeV positron beam accelerated by cyclotron HM12 (Sumitomo Heavy Industries, Ltd.) to [¹⁸O]H₂O with isotope purity 95% or greater. Then, ¹⁸F⁻ was captured on the resin by passing the solution through the anion exchange resin (AG1-X8), and eluted with 33mM of K₂CO₃ solution. 300µl of this K₂CO₃ aqueous solution containing ¹⁸F - (3.57 GBq) was taken in the brown vial (volume, 10mL). To this solution, Kryptofix222 (16mg) and acetonitrile (2.0mL) were added. It was, being heated in the oil bath (110°C), sprayed with He gas, water being azeotroping to completely remove acetonitrile. Furthermore, acetonitrile was added and similar operation to remove acetonitrile under the heating condition was repeated three times to make within the vial in an anhydrous state. To this, DMSO solution (0.7 ml) dissolving THK-857 (3.5 mg), the labeling precursor, was added and heated and stirred in the oil bath (110°C) for 10 minutes. Then, the reaction solution was diluted with distilled water (7 ml), and loaded on the Sep-Pak tC18 cartridge (Waters). After washing the cartridge with distilled water, the solution eluted with EtOH was added on semipreparative HPCL (column: Inertsil[R] ODS-3 (10×250 mm), transfer phase: MeCN/20mM NaH₂PO₄ = 40/60, flow rate: 7.0 mL/min) to fractionate the radiation peak derived from [¹⁸F] THK-837 which is to be eluted at about 21 minutes. The radiochemical yield after decay correction obtained from the radiation of the fraction was 26%.

### Labeling synthesis of [¹⁸F] THK-853

¹⁸F⁻ was synthesized by radiating 12 MeV proton beam accelerated by cyclotron HM12 (Sumitomo Heavy Industries, Ltd.) to [¹⁸O]H₂O with isotope purity 95% or greater. Then, ¹⁸F⁻ was captured on the resin by passing the solution through the anion exchange resin (AG1-X8), and eluted with 33mM of K₂CO₃ solution. 300µl of this K₂CO₃ aqueous solution containing ¹⁸F⁻ (3.85 GBq) was taken in the brown vial (volume, 10mL). To this solution, Kryptofix222 (16mg) and acetonitrile (2.0mL) were added. It was, being heated in the oil bath (110°C), sprayed with He gas, water being azeotroping to completely remove acetonitrile. Furthermore, acetonitrile was added and similar operation to remove acetonitrile under the heating condition was repeated three times to make within the vial in an anhydrous state. To this, DMSO solution (0.7 ml) dissolving THK-852 (3.5 mg), the labeling precursor, was added and heated and stirred in the oil bath (110°C) for 10 minutes. Then, the reaction solution was diluted with distilled water (7 ml), and loaded on the Sep-Pak tC18 cartridge (Waters). After cleaning the cartridge with distilled water, the solution eluted with EtOH was added on semipreparative HPCL (column: Inertsil® ODS-3 (10×250 mm), transfer phase: MeCN/20mM NaH₂PO₄ = 45/55, flow speed: 7.0 mL/min) to fractionate the radiation peak derived from [¹⁸F] THK-853 which is to be eluted at about 22 minutes. The radiochemical yield after decay correction obtained from the radiation of this fraction was 29%.

### Preparation of a physiological saline containing the labeled compound

Prepared HPLC fraction containing [¹⁸F] THK-837 or [¹⁸F] THK-853 obtained from the labeling synthesis was diluted with the distilled water (about 20 mL), loaded on the Sep-Pak tC18 cartridge (Waters), and after washing the cartridge with the distilled water (10 mL), the labeled compound was eluted with EtOH (4 to 5 mL). To this EtOH eluate, an appropriate amount of 5% Polysorbate 80 ethanol solution was added, and while it was heated at 80 °C, the solvent was distilled off with the rotary evaporator. The physiological saline solution containing the labeled compound was prepared by dissolving the mixture of the obtained labeled compound and Polysorbate 80 in the physiological saline solution. The radiochemical purity of the drug solution after preparation was 95% or more.

### The evaluation of blood-brain barrier permeability of the mouse and osseous accumulation of the labeled compound

The physiological saline solution containing [¹⁸F] BF-837 or [¹⁸F] THK-853 was administered into the tail vein of the male ICR mice (6-7 weeks of age), and blood-brain barrier permeability and osseous accumulation of each labeled compound were evaluated based on accumulation of radioactive in the brain tissue and the bone tissue at 2, 30, 60 minutes after.
Since BF-227 (2-[2-(2-dimethylaminothiazole-5-yl) ethenyl]-6-(2-fluoroethoxy) benzoxazole) is comparatively analogous in its chemical structure to the compounds of the present invention, and although BF-227 has been used as [¹¹C] labeling compound, it has F in the chemical structure (Kudo et al., Journal of Nuclear Medicine 48, 553-561, 2007), therefore, in the experiment, the difference in the feature between [¹⁸F] BF-227as the PET probe for diagnosis of Alzheimer's disease and [¹⁸F] labeled compound of the present invention was examined.
The radiochemical purity of the used physiological saline containing the labeled compound used was 95% or greater, and specific activity was 18.5 to 148 GBq/µmol, and 1.11 - 2.22 MBq of the labeled compound per mouse was administered. In the evaluation of radiation accumulation, the ratio of radiation per unit weight of the evaluation tissue against the whole radiation administered (% Injected Dose/g of tissue; %ID/g) was used as the index. For measurement of the radiation, the gamma counter (1480 WIZARD, PerkinElmer, Inc.) was used. The experimental procedure was as follows. The labeled compound was administered into the caudal vein and dislocation in the cervical spine of the mice was conducted under the ether anesthesia at 2, 30, and 60 minutes after, and after promptly harvesting the blood from the heart, the whole brain (including the cerebellum, brain stem) and the femur were removed. Then the radiation and tissue weight of each sample were measured and %ID/g was calculated using these data. The results of this evaluation experiment are shown in Table 2.

**[Table 2]**

| | | %ID/g or ml (mean±standard deviation) | | |
|---|---|---|---|---|
| | | 2 minutes | 30 minutes | 60 minutes |
| [¹⁸F]THK- 837 | Brain | 6.54±0.19 | 0.94±0.08 | 0.43±0.03 |
| | Blood plasma | 3.30±0.17 | 1.73±0.26 | 1.35±0.77 |
| | Bone | 1.80±0.17 | 1.27±0.11 | 1.64±0.12 |
| [¹⁸F]THK- 853 | Brain | 7.56±0.87 | 1.31±014 | 0.59±0.04 |
| | Blood plasma | 2.81±0.37 | 2.11±0.50 | 1.59±0.12 |
| | Bone | 1.64±0.19 | 0.98±0.20 | 0.96±0.04 |
| [¹⁸F]BF-227 | Brain | 6.05±0.45 | 1.91±0.05 | 1.67±0.14 |
| | Blood plasma | 2.93±0.08 | 2.14±0.17 | 2.09±0.15 |
| | Bone | 1.59±0.27 | 4.38±1.24 | 7.04±0.75 |

The blood-brain barrier permeability of the labeled compounds for PET or for SPECT targeting the central nerve system is considered to be sufficient if it is 0.5% ID/g or greater. From the standpoint, [¹⁸F] labeled compound of the present invention is [¹⁸F] labeled compound with extremely high permeability to the brain.
With regard to [¹⁸F] labeled compound, what becomes frequently problematic is osseous accumulation of ¹⁸F ion based on defluorination in vivo (Tipre et al., Journal of Nuclear Medicine 47 pp345-353 2006). Since ¹⁸F ion has high osseous accumulation, if [¹⁸F] labeled compound subjected to defluorination is used as the diagnostic probe for Alzheimer's disease, the PET image obtained will be an image of the bone (cranial bone). However, at present, it cannot be said that there are labeled compounds less subjected to defluorination to the satisfactory degree (refer to Cai et al., Journal of Medicinal Chemistry 47, 2208-2218, 2224; Zhang et al., Journal of Medicinal Chemistry 48, pp5980-5988, 2005; Chang et al., Nuclear Medicine and Biology 33, 811-820, 2006; and Stephenson et al., Bioconjugate Chemistry 18, 238-246, 2007).
The results of osseous accumulation of [¹⁸F] THK-837 and [¹⁸F] THK-853 simultaneously measured at the time of evaluation on transfer to the brain are shown in table 2. Osseous accumulation was not observed in [¹⁸F] THK -837 and [¹⁸F] THK-853, on the other hand, time dependence marked accumulation was observed after administration of [¹⁸F] BF-227. The above suggested that in vivo, [¹⁸F] THK-837 and [¹⁸F] THK-853 are not subjected to defluorination, while [¹⁸F] BF-227 is easily subjected to defluorination, and ¹⁸F ion is accumulated in bones.

The summary of the results of the above experiment shows that the [¹⁸F] labeled compounds of the present invention, in particular, [¹⁸F] THK -837 and [¹⁸F] THK-853 are clearly more excellent than [¹⁸F]. BF-227 in wash-out from the brain (Table 2). Osseous accumulation of ¹⁸F ion based on defluorination as observed in [¹⁸F] BF-227 is scarcely observed in the [¹⁸F] labeled compound of the present invention, in particular, [¹⁸F] THK -837 and [¹⁸F] THK-853. Thus, it is confirmed that defluorination is suppressed to the satisfactory degree in these compounds (Table 2).
Based on the above, from the viewpoint of PET probe for diagnosis of Alzheimer's disease, [¹⁸F] labeled compounds of the present invention, in particular, [¹⁸F] THK -837 and [¹⁸F] THK-853 have extremely higher usefulness when compared with [¹⁸F] BF-227 which has no structure of the compound of the present invention.

### INDUSTRIAL APPLICABILITY OF THE INVENTION

The present invention provides the compound for a diagnostic probe for conformation disease, in particular to an imaging diagnostic probe, the pharmaceutical composition comprising the compound for prevention and/or treatment of conformation disease, etc. The present invention is very useful in the early discovery, treatment and prevention of conformation diseases such as Alzheimer disease. The present invention can also be used in the production of diagnosing agents and kits for conformation diseases, the production of agents for treatment and prevention of conformation diseases, and the study of conformation diseases, etc.

## Claims

1. A compound selected from the group consisting of formula (I): 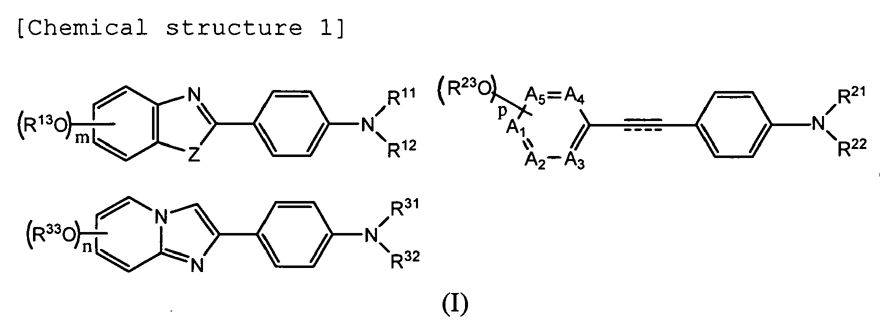 and
[wherein any one of R11, R12, or R13 is a group represented by formula (II):
[Chemical structure 2] -X-F (II)
(wherein X is an alkyl group of 3 through 10 carbons substituted by 1 through 3 hydroxy groups), and
remaining two of R11, R12 or R13 are hydrogen atoms or lower alkyl groups, or
in case that R13 is a group represented by above-mentioned formula (II), R11, R12 and nitrogen atoms bonded thereto, together, form 3-8 membered nitrogen-containing aliphatic cyclic group,
m is 1, or in case that either of R11 or R12 is a group represented by above-mentioned formula (II), m may be 0,
Z represents an oxygen atom or sulfur atom;
any one of R21, R22 or R23 is a group represented by formula (II):
[Chemical structure 3] -X-F (II)
(wherein X has the above-mentioned meaning), and
remaining two of R21, R22 or R23 are hydrogen atoms or lower alkyl groups, or
in case that R23 is a group represented by above-mentioned formula (II), R21, R22 and nitrogen atoms bonded thereto, together, form 3-8 membered nitrogen-containing aliphatic cyclic group,
p is 1, or in case that either of R21 or R22 is a group represented by above-mentioned formula (II), p may be 0, and 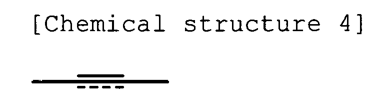 represents 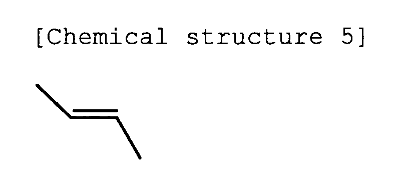 or 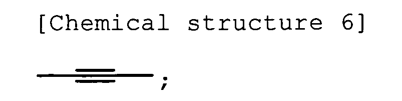 and one of R31, R32 or R33 is a group represented by formula (II):
[Chemical structure 7] -X-F (II)
(wherein X has the above-mentioned meaning), and
remaining two of R31, R32 or R33 are hydrogen atoms or lower alkyl groups, or
in case that R33 is a group represented by above-mentioned formula (II), R31, R32 and nitrogen atoms bonded thereto, together, form 3-8 membered nitrogen-containing aliphatic cyclic group,
n is 1, or in case that either of R31 or R32 is a group represented by above-mentioned formula (II), n may be 0, and
one or two of A1 through A5 is a nitrogen atom and the remaining three or four are CH], pharmaceutically acceptable salt or solvate thereof.

2. The compound according to claim 1, pharmaceutically acceptable salt or solvate thereof, wherein X is a straight-chain or branched alkyl group of 3 through 7 carbons substituted by 1 or 2 hydroxy groups.

3. The compound according to claim 1, pharmaceutically acceptable salt or solvate thereof, wherein formula (II) is a group selected from the group consisting of formula (III): 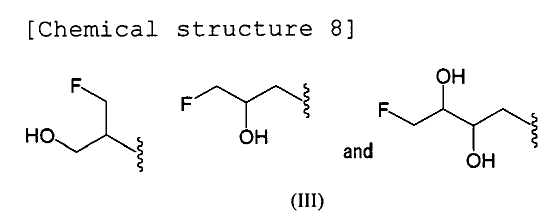 wherein, 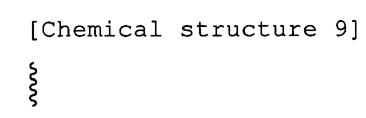 shows the bonded portion with an oxygen atom or nitrogen atom.

4. The compound according to claim 1, pharmaceutically acceptable salt or solvate thereof, wherein the compound represented by formula (I) is a compound expressed by formula (I-1): 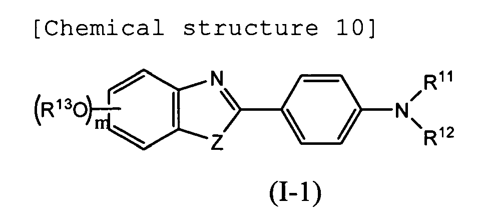 wherein any one of R11, R12, or R13 is a group represented by formula (II):
[Chemical structure 11] X-F (II)
(wherein X is an alkyl group of 3 through 10 carbons substituted by 1 through 3 hydroxy groups), and is selected from consisting of formula (III): 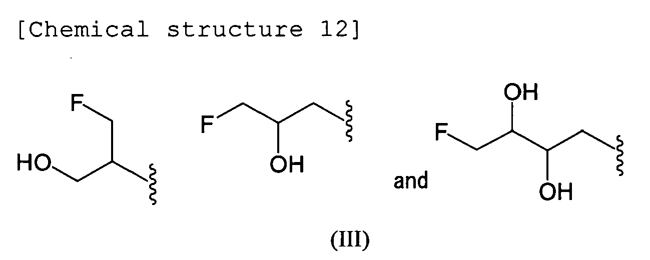 (wherein, 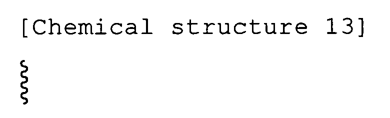 shows the bonded portion with an oxygen atom or nitrogen atom),
remaining two of R11, R12 or R13 are hydrogen atoms or lower alkyl groups, or
in case that R13 is a group represented by above-mentioned formula (II), R11, R12 and nitrogen atoms bonded thereto, together, form 3-8 membered nitrogen-containing aliphatic cyclic group,
m is 1, or in case that either of R11 or R12 is a group represented by above-mentioned formula (II), m may be 0, and
Z represents an oxygen atom or sulfur atom.

5. The compound according to claim 4, pharmaceutically acceptable salt or solvate thereof, wherein R13 is a group selected from the group consisting of formula (III): 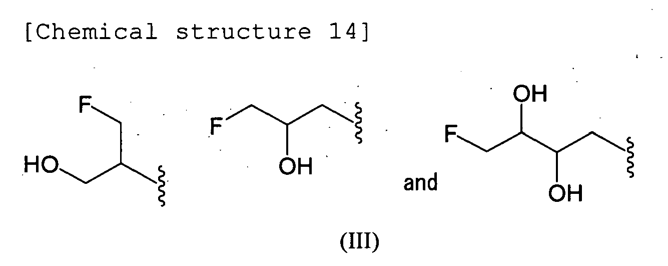 (wherein, 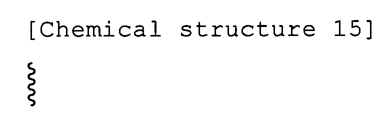 shows the bonded portion with an oxygen atom or nitrogen atom),
and R11 and R12 are independently hydrogen atoms or low alkyl groups, or R11, R12 and nitrogen atoms bonded thereto, together, form 3-8 membered nitrogen-containing aliphatic cyclic group, m is 1, and
Z is a sulfur atom.

6. The compound according to claim 5, pharmaceutically acceptable salt or solvate thereof, wherein R13 is a group represented by formula (III-1): 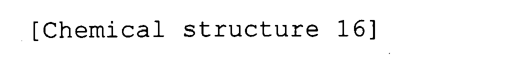 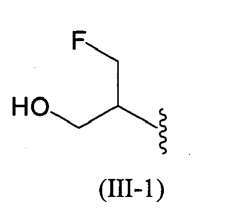 wherein, 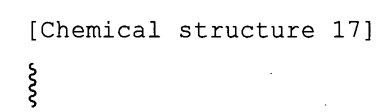 shows the bonded portion with an oxygen atom or nitrogen atom.

7. The compound according to claim 1, pharmaceutically acceptable salt or solvate thereof, wherein formula (I) is a compound expressed by formula (I-2): 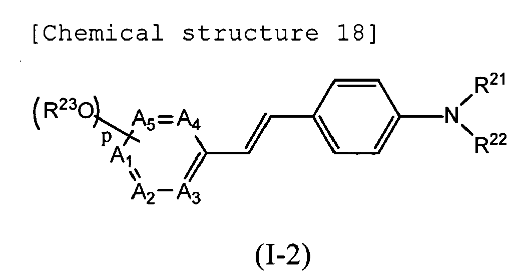 wherein any one of R22 or R23 is a group represented by formula (II):
[Chemical structure 19] -X-F (II)
(wherein X is an alkyl group of 3 through 10 carbons substituted by 1 through 3 hydroxy groups), and
remaining two of R21, R22 or R23 are hydrogen atoms or lower alkyl groups, or
in case that R23 is a group represented by formula (II):
[Chemical structure 20] -X-F (II)
(wherein X has the above-mentioned meaning), R21, R22 and nitrogen atoms bonded thereto, together, form 3-8 membered nitrogen-containing aliphatic cyclic group,
p is 1, or in case that either of R21 or R22 is a group represented by above-mentioned formula (II), p may be 0, and
one or two of A1 through A5 is a nitrogen atom and the remaining three or four are CH.

8. The compound according to claim 7, pharmaceutically acceptable salt or solvate thereof, wherein R23 is a group selected from the group consisting of formula (III): 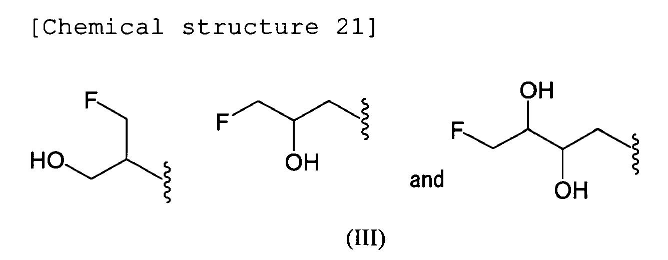 (wherein, 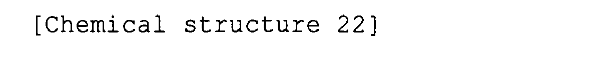 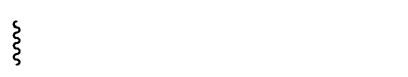 shows the bonded portion with an oxygen atom or nitrogen atom),
and R21 and R22 are independently hydrogen atoms or low alkyl groups, or R21, R22 and nitrogen atoms bonded thereto, together, form 3-8 membered nitrogen-containing aliphatic cyclic group, and
p is 1.

9. The compound according to claim 8, pharmaceutically acceptable salt or solvate thereof, wherein R23 is a group represented by formula (III-1): 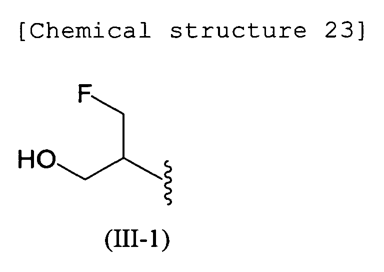 formula (III-1).

10. The compound according to claim 1, pharmaceutically acceptable salt or solvate thereof, wherein the compound represented by formula (I) is either 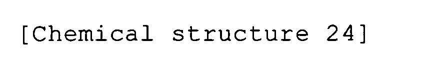 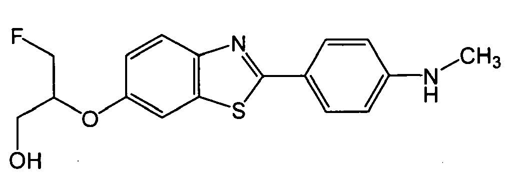 or

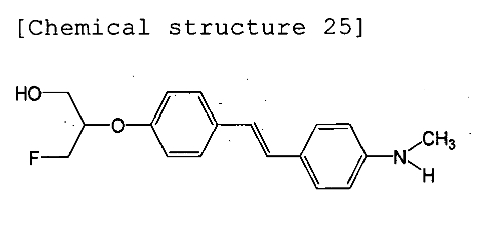

11. The compound according to any one of claims 1 to 10, pharmaceutically acceptable salt or solvate thereof, which is labeled.

12. The compound according to claim 11, pharmaceutically acceptable salt or solvate thereof, wherein the label is a radionuclide.

13. The compound according to claim 12, pharmaceutically acceptable salt or solvate thereof, wherein the radionuclide is a gamma-ray emitting radionuclide.

14. The compound according to claim 11, pharmaceutically acceptable salt or solvate thereof, wherein the label is a positron emitter.

15. The compound according to claim 14, pharmaceutically acceptable salt or solvate thereof, wherein the positron emitter is selected from the group consisting of ¹¹C, 1³N, ¹⁵O, ¹⁸F, ³⁵mCl, ⁷⁶Br, ⁴⁵Ti, ⁴⁸V, ⁶⁰Cu, ⁶¹Cu, ⁶²Cu, ⁶⁶Ga, ⁸⁹Zr, ⁹⁴mTc and ¹²⁴I.

16. The compound according to claim 14, pharmaceutically acceptable salt or solvate thereof, wherein the positron emitter is ¹¹C or ¹⁸F.

17. A pharmaceutical composition comprising the compound according to any one of claims 1 to 16, pharmaceutically acceptable salt or solvate thereof.

18. A pharmaceutical composition comprising the compound according to any one of claims 1 to 16, pharmaceutically acceptable salt or solvate thereof, and a solubilizing agent.

19. The pharmaceutical composition according to claim 18, wherein the solubilizing agent is selected from the group consisting of polysolvate 80, polyethylene glycol, ethanol, and propylene glycol.

20. The pharmaceutical composition according to any one of claims 17 to 19, which is an injection drug.

21. A composition for diagnosis of conformation diseases comprising the compound according to any one of claims 1 to 16, pharmaceutically acceptable salt or solvate thereof.

22. A pharmaceutical composition to treat and/or prevent conformation diseases, comprising the compound according to any one of claims 1 to 16, pharmaceutically acceptable salt or solvate thereof.

23. A kit for diagnosis of conformation diseases, comprising the compound according to any one of claims 1 to 16, pharmaceutically acceptable salt or solvate thereof as a essential component.

24. A composition or kit for detecting or staining proteins with beta sheet structure or neurofibrillary tangle, comprising the compound according to any one of claims 1 to 16, pharmaceutically acceptable salt or solvate thereof as a essential component.

25. The kit according to claim 23 or 24 for diagnostic imaging.

26. A method for treatment and/or prevention of conformation diseases in a subject, whichcomprises administering the compound according to any one of claims 1 to 16, pharmaceutically acceptable salt or solvate thereof to the subject.

27. A method for diagnosis of conformation diseases in a subject, which comprises administering the compound according to any one of claims 1 to 16, pharmaceutically acceptable salt or solvate thereof to the subject.

28. Use of the compound according to any one of claims 1 to 16, pharmaceutically acceptable salt or solvate thereof for producing a composition or kit of aid diagnosis of conformation diseases in a subject.

29. The use of the compound according to any one of claims 1 to 16, pharmaceutically acceptable salt or solvate thereof for producing a pharmaceutical composition for treatment and/or prevention of conformation diseases in a subject.

30. A method for detecting or staining proteins with beta sheet structure or neurofibrillary tangle in a sample, which comprises using the compound according to any one of claims 1 to 16, pharmaceutically acceptable salt or solvate thereof to stain samples.

31. Use of the compound according to any one of claims 1 to 16, pharmaceutically acceptable salt or solvate thereof for producing a composition or a kit for detecting or staining proteins with beta sheet structure or neurofibrillary tangle in a sample.

32. A compound selected from the group consisting of formula (I'): 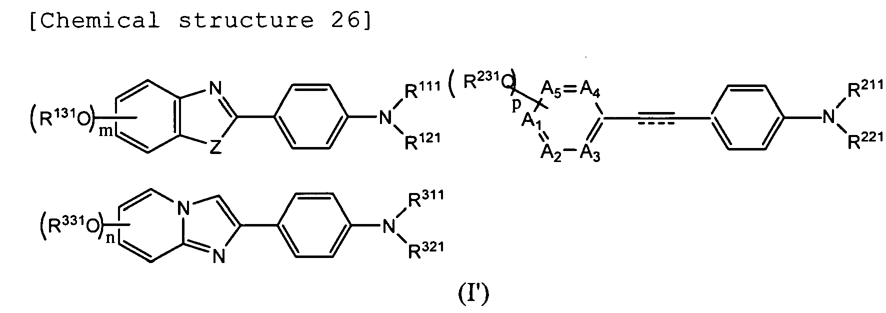 and
wherein any one of R111, R121, or R131 is a group represented by formula (II'):
[Chemical structure 27] -X-OTs (II')
(wherein X is an alkyl group of 3 through 10 carbons substituted by 1 through 3 hydroxy groups), and
remaining two of R111, R121 or R131 are hydrogen atoms or lower alkyl groups, or
in case that R131 is a group represented by above-mentioned formula (II), R111, R121 and nitrogen atoms bonded thereto, together, form 3-8 membered nitrogen-containing aliphatic cyclic group,
m is 1, or in case that either of R111 or R121 is a group represented by above-mentioned formula (II'), m may be 0,
Z represents an oxygen atom or sulfur atom,
any one of R21, R22 or R23 is a group represented by formula (II') comprising:
[Chemical structure 28] -X-OTs (II')
(wherein X has the above-mentioned meaning), and
remaining two of R211, R221 or R231 are hydrogen atoms or lower alkyl groups, or
in case that R231 is a group represented by above-mentioned formula (II'), R211, R221 and nitrogen atoms bonded thereto, together, form 3-8 membered nitrogen-containing aliphatic cyclic group,
n is 1, or in case that either of R211 or R221 is a group represented by above-mentioned formula (II'), n may be 0,
and 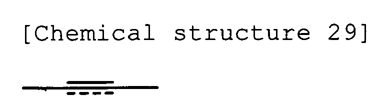 represents 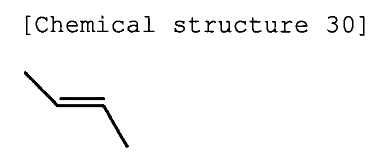 or 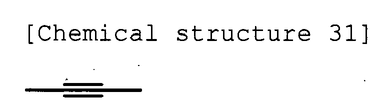 and any one of R311, R321 or R331 is a group represented by formula (II'):
[Chemical structure 32] -X-OTs (II')
(wherein X has the above-mentioned meaning), and
remaining two of R311, R321 or R331 are hydrogen atoms or lower alkyl groups, or
in case that R331 is a group represented by above-mentioned formula (II'), R311, R321 and nitrogen atoms bonded thereto, together, form 3-8 membered nitrogen-containing aliphatic cyclic group,
p is 1, or in case that either of R31 or R32 is a group represented by above-mentioned formula (II'), p may be 0, and
one or two of A1 through A5 is a nitrogen atom and the remaining three or four are CH.

33. The compound according to claim 32, wherein the group represented by formula (II') is selected from the group consisting of formula (III'): 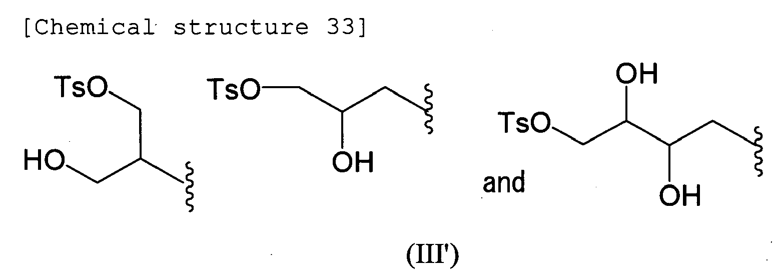 wherein, 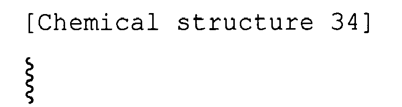 shows the bonded portion with an oxygen atom or nitrogen atom.

34. The compound according to (32), wherein the compound represented by formula (I') is either 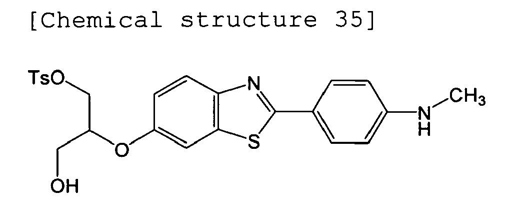 or
